(19) Europäisches Patentamt
European Patent Office
Office européen des brevets

(11) EP 0 393 604 B1

# (12) EUROPEAN PATENT SPECIFICATION

(45) Date of publication and mention of the grant of the patent:
**29.12.1997 Bulletin 1997/52**

(51) Int Cl.6: **C07D 471/04**, A61K 31/55
// (C07D471/04, 243:00, 221:00)

(21) Application number: **90107293.4**

(22) Date of filing: **18.04.1990**

## (54) 6,11-Dihydro-5H-pyrido(2,3-b)(1,5,)benzodiazepin-5-ones and thiones and their use in the prevention or treatment of AIDS

6,11-Dihydro-5H-pyrido(2,3-b)(1,5)benzodiazepin-5-one und Thione und ihre Verwendung für die Vorbeugung oder Behandlung von AIDS

6,11-Dihydro-5H-pyrido(2,3-b)(1,5)benzodiazépine-5-ones et thiones et leur utilisation pour la prévention ou le traitement du SIDA

(84) Designated Contracting States:
**AT BE CH DE DK ES FR GB GR IT LI LU NL SE**

(30) Priority: **20.04.1989 US 340973**
**28.06.1989 US 372732**
**17.11.1989 US 438570**

(43) Date of publication of application:
**24.10.1990 Bulletin 1990/43**

(73) Proprietors:
- **BOEHRINGER INGELHEIM PHARMACEUTICALS INC.**
  **Ridgefield, Connecticut 06877-0368 (US)**
- **Dr. Karl Thomae GmbH**
  **D-88397 Biberach (DE)**

(72) Inventors:
- **Hargrave, Karl D., Dr.**
  **Brookfield, Connecticut 06805 (US)**
- **Schmidt, Günther, Dr.**
  **(DE)**
- **Engel, Wolfhard, Dr.**
  **D-7950 Biberach 1 (DE)**
- **Trummlitz, Günter, Dr.**
  **D-7951 Warthausen (DE)**
- **Eberlein, Wolfgang, Dr.**
  **D-7950 Biberach 1 (DE)**

(74) Representative: **Laudien, Dieter, Dr. et al**
**c/o Boehringer Ingelheim GmbH**
**55216 Ingelheim (DE)**

(56) References cited:
**EP-A- 0 344 543**
**GB-A- 1 456 627**
**GB-A- 1 538 366**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Field of the Invention

The invention relates to novel 6,11-dihydro-5H-pyrido[2,3-b]-[1,5]benzodiazepin-5-ones and -thiones, methods for preparing these compounds, the use of these and related but known compounds in the prevention or treatment of AIDS, and to pharmaceutical compositions containing these compounds.

### Background of the Invention

The human disease, Acquired Immune Deficiency Syndrome (AIDS), is caused by the Human Immunodeficiency Virus (HIV), particularly the strain known as HIV-1.

Like other viruses, HIV-1 cannot replicate without commandeering the biosynthetic apparatus of the host cell it infects. It causes this apparatus to produce the structural proteins which make up the viral progeny. These proteins are coded for by the genetic material contained within the infecting virus particle, or virion. Being a retrovirus, however, the genetic material of HIV is RNA, not DNA as in the host cell's genome. Accordingly, the viral RNA must first be converted into DNA, and then integrated into the host cell's genome, in order for the host cell to produce the required viral proteins.

The conversion of the RNA to DNA is accomplished through the use of the enzyme reverse transcriptase (RT), which is included within the infecting virion along with the RNA. Reverse transcriptase has three enzymatic functions; it acts as an RNA-dependent DNA polymerase, as a ribonuclease, and as a DNA-dependent DNA polymerase. Acting first as a RNA-dependent DNA polymerase, RT makes a single-stranded DNA copy of the viral RNA. Next, acting as a ribonuclease, RT frees the DNA just produced from the original viral RNA and then destroys the original RNA. Finally, acting as a DNA-dependent DNA polymerase, RT makes a second complementary, DNA strand, using the first DNA strand as a template. The two strands form double-stranded DNA, the form of DNA found in the host cell's genome, which is integrated into the host cell's genome by another enzyme, called an integrase.

Compounds which inhibit the enzymatic functions of HIV-1 reverse transcriptase will inhibit replication of HIV-1 in infected cells. Such compounds are useful in the prevention or treatment of HIV-1 infection in human subjects.

### Summary of the Invention

A first aspect of the invention comprises novel 6,11-dihydro-5H-pyrido[2,3-b][1,5]benzodiazepin-5-ones and -thiones. These possess inhibitory activity against HIV-1 RT. A second aspect of the invention comprises methods for making these novel compounds. A third aspect of the invention is a method for preventing or treating HIV-1 infection which comprises administering, to a human being exposed to or infected by HIV-1, a prophylactically or therapeutically effective amount of one of the above-mentioned novel compounds. A fourth aspect of the invention is a method for preventing or treating HIV-1 infection which comprises administering certain known 6,11-dihydro-5Hpyrido[2,3-b][1,5]-benzodiazepin-5-ones. These compounds also possess inhibitory activity against HIV-1 RT. Fifth and sixth aspects of the invention comprise novel 5-thione analogues of the above-mentioned known 5-ones, and a method for their preparation. A seventh aspect of the invention comprises a method for preventing or treating HIV-1 infection which comprises administering one or more of these novel 5-thione analogues. A final aspect of the invention comprises pharmaceutical compositions suitable for the prevention or treatment of HIV-1 infection comprising the above-mentioned compounds, both novel and known.

### Detailed Description of the Invention

In one of its composition of matter aspects, the invention comprises 6,11-dihydro-5H-pyrido[2,3-b][1,5]benzodiazepin-5-ones and -thiones of the formula 1

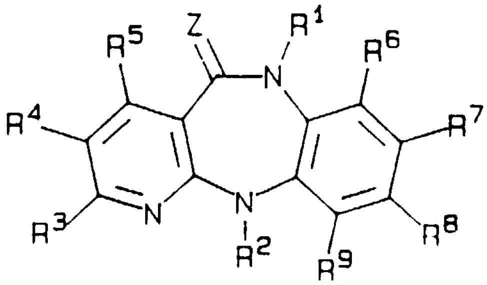

(1)

wherein,
Z is oxygen or sulfur;

$R^1$ is hydrogen, alkyl or fluoroalkyl of 1 to 5 carbon atoms, cyclopropyl, alkenyl or alkynyl of 2 to 5 carbon atoms, 2-halo-propen-1-yl, arylmethyl (wherein the aryl moiety is thienyl, thienyl or furanyl, which is either unsubstituted or substituted by methyl, methoxy or halogen), alkanoyl of 2 to 3 carbon atoms, or alkoxyalkyl or alkylthioalkyl of 2 to 4 carbon atoms;

$R^2$ is alkyl or fluoroalkyl of 1 to 5 carbon atoms, cycloalkyl of 3 to 5 carbon atoms, alkenyl or alkynyl of 2 to 5 carbon atoms, alkoxyalkyl or alkylthioalkyl of 2 to 4 carbon atoms, alkanoyl of 2 to 4 carbon atoms, hydroxyalkyl of 2 to 5 carbon atoms, arylmethyl (wherein the aryl moiety is phenyl, thienyl or furanyl, which is either unsubstituted or substituted by alkyl or alkoxy of 1 to 3 carbon atoms, hydroxyl, or halogen), phenyl (which is either unsubstituted or substituted by alkyl or alkoxy of 1 to 3 carbon atoms, halogen or hydroxyl) or alkoxy-carbonylmethyl wherein the alkoxy moiety contains 1 to 5 carbon atoms;

$R^3$, $R^4$, and $R^5$ are each independently hydrogen or alkyl of 1 to 3 carbon atoms, with the proviso that at least one of these substituents is hydrogen; or,

one of $R^3$, $R^4$ and $R^5$ is butyl, alkanoyl of 1 to 3 carbon atoms, hydroxyalkyl of 1 to 4 carbon atoms, alkoxycarbonyl of 2 to 3 carbon atoms, alkoxycarbonylalkyl wherein both the alkoxy and alkyl moieties contain 1 to 2 carbon atoms, halogen, trihalomethyl, hydroxyl, alkoxy of 1 to 3 carbon atoms, alkythio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido or mono- or di-alkylaminoalkyl wherein the alkyl moieties each contain 1 to 2 carbon atoms, and the remaining two substituents are hydrogen or methyl; or,
when Z is oxygen, one of $R^3$, $R^4$ and $R^5$ is alkylsulfinyl or alkylsulfonyl of 1 to 3 carbon atoms, with the proviso that the remaining two substituents are hydrogrogen or methyl; and,
$R^6$, $R^7$, $R^8$ and $R^9$ are each hydrogen; or,
one of $R^6$, $R^7$, $R^8$ and $R^9$ is alkyl of 1 to 4 carbon atoms, alkanoyl of 1 to 3 carbon atoms, alkoxycarbonyl of 2 to 3 carbon atoms, hydroxyalkyl of 1 to 4 carbon atoms, alkoxycarbonylalkyl wherein both the alkoxy and alkyl moieties contain 1 to 2 carbon atoms, halogen, trihalomethyl, hydroxyl, alkoxy of 1 to 3 carbon atoms, alkylthio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxylalkyl of 2 to 3 carbon atoms, cyano, nitro, carboxyl, carbamyl, amino, azido or mono- or di-alkylaminoalkyl wherein each alkyl moiety contains 1 to 2 carbon atoms, and the remaining three substituents are hydrogen or two of the remaining three substituents are hydrogen and one is methyl, ethyl or halogen.

A subgeneric aspect of the invention comprises compounds of formula I, wherein,
Z is oxygen or sulfur;
$R^1$ is hydrogen, alkyl of 1 to 4 carbon atoms, alkenyl or alkynyl of 2 to 4 carbon atoms, 2-halo-propen-1-yl, or alkoxyalkyl or alkylthioalkyl of 2 to 3 carbon atoms;
$R^2$ is alkyl of 1 to 4 carbon atoms, cycloalkyl of 3 to 4 carbon atoms, alkenyl or alkynyl of 2 to 4 carbon atoms, alkoxyalkyl or alkylthioalkyl of 2 to 4 carbon atoms, alkanoyl of 2 to 3 carbon atoms, hydroxyalkyl of 2 to 4 carbon atoms, arylmethyl (wherein the aryl moiety is phenyl or thienyl, which is either unsubstituted or substituted by methyl, methoxy, hydroxyl

or halogen), phenyl (which is either unsubstituted or substituted by methyl, methoxy, hydroxyl or halogen) or alkoxycarbonylmethyl wherein the alkoxy moiety contains 1 to 5 carbon atoms;
$R^3$, $R^4$, and $R^5$ are each independently hydrogen or methyl, with the proviso that at least one of these substituents is hydrogen, or $R^5$ is ethyl, propyl or butyl with the other two substituents being hydrogen;
$R^6$ is hydrogen, methyl or ethyl with the proviso that $R^7$ is hydrogen or methyl;
$R^7$ is alkyl of 1 to 3 carbon atoms, alkanoyl of 1 to 3 carbon atoms, alkoxycarbonyl of 1 to 3 carbon atoms, hydroxyalkyl of 1 to 3 carbon atoms, alkoxycarbonylalkyl wherein the alkoxy and alkyl moieties each contain 1 to 2 carbon atoms, halogen, trifluoromethyl, hydroxyl, alkoxy or alkylthio of 1 to 2 carbon atoms, acetyloxy, alkanoylamino or aminoalkyl of 1 to 2 carbon atoms, cyano, nitro, amino, or mono- or di-methyl or -ethylamino, with the proviso that $R^8$ is hydrogen;
$R^8$ is alkyl of 1 to 3 carbon atoms, alkanoyl of 1 to 3 carbon atoms, alkoxycarbonyl of 1 to 3 carbon atoms, hydroxyalkyl of 1 to 3 carbon atoms, alkoxycarbonylalkyl wherein the alkoxy and alkyl moieties each contain 1 to 3 carbon atoms, halogen, trifluoromethyl, hydroxyl, alkoxy or alkylthio of 1 to 2 carbon atoms, acetyloxy, alkanoylamino or aminoalkyl of 1 to 2 carbon atoms, cyano, nitro, amino, or mono- or di-methyl or -ethylamino with the proviso that $R^7$ is hydrogen; or, when Z is oxygen and $R^8$ is hydrogen or methyl, $R^7$ may additionally be alkylsulfinyl or alkylsulfonyl of 1 to 2 carbon atoms, and when Z is oxygen and $R^7$ is hydrogen or methyl, $R^8$ may additionally be alkylsulfinyl or alkylsulfonyl of 1 to 2 carbon atoms; or,
$R^7$ and $R^8$ are both hydrogen, methyl or halogen; and,
$R^9$ is hydrogen or methyl with the proviso that $R^8$ is hydrogen or methyl.

A further subgeneric aspect of the invention comprises compounds of formula I, wherein,
Z is oxygen or sulfur;
$R^1$ is hydrogen, alkyl of 1 to 3 carbon atoms, 2-halo-2-propen-1-yl, or alkoxyalkyl or alkylthioalkyl of two to three carbon atoms;
$R^2$ is alkyl of 1 to 4 carbon atoms, cycloalkyl of 3 to 4 carbon atoms, alkenyl or alkynyl of 2 to 4 carbon atoms, alkyloxyalkyl or alkylthioalkyl of 2 to 3 carbon atoms, alkanoyl of 2 to 3 carbon atoms, hydroxyalkyl of 2 to 4 carbon atoms, arylmethyl (wherein the aryl moiety is phenyl or thienyl, which is either unsubstituted or substituted by methyl, methoxy or halogen) or alkoxycarbonylmethyl wherein the alkoxy moiety contains 1 to 5 carbon atoms; and,
$R^3$ through $R^9$ are as set forth below in Table A.

TABLE A

| | $R^3$ | $R^4$ | $R^5$ | $R^6$ | $R^7$ | $R^8$ | $R^9$ |
|---|---|---|---|---|---|---|---|
| a | H | H | H | H | H | $CF_3$ | H |
| b | H | H | H | H | Cl | H | H |
| c | H | H | H | H | $CH_3$ | $CH_3$ | H |
| d | $CH_3$ | H | H | H | $CH_3$ | $CH_3$ | H |
| e | $CH_3$ | $CH_3$ | H | H | $CH_3$ | $CH_3$ | H |
| f | H | H | H | H | $CH_3$ | $CH_3$ | H |
| g | H | H | H | H | H | Cl | H |
| h | H | H | H | H | H | H | H |
| i | H | H | H | $CH_3$ | H | H | H |
| j | H | H | H | H | $CH_3O_2C$- | H | H |
| k | H | H | H | H | $C_2H_5O_2C$- | H | H |
| l | H | H | H | H | NC- | H | H |
| m | H | H | H | H | $CH_3CO$- | H | H |
| n | H | H | H | H | H | $CH_3O_2C$- | H |
| o | H | H | H | H | H | $C_2H_5O_2C$- | H |
| p | H | H | H | H | H | NC- | H |
| q | H | H | H | H | H | $CH_3CO$- | H |

A more particular subgeneric aspect of the invention comprises compounds of formula I wherein,
Z is oxygen or sulfur;
$R^1$ is hydrogen, alkyl of 1 to 2 carbon atoms or allyl;
$R^2$ is alkyl of 2 to 3 carbon atoms, cyclopropyl or allyl; and
$R^3$ through $R^9$ are each hydrogen, or $R^7$ and $R^8$ are both methyl or chloro and $R^3$, $R^4$, $R^5$, $R^6$ and $R^9$ are each hydrogen.

Compounds of Formula I may, if desired, be converted into their non-toxic, pharmaceutically acceptable acid addition salts by conventional methods; for example, by dissolving a compound of formula I in a suitable solvent and

acidifying the solution with one of more molar equivalents of the desired acid. The invention also comprises such salts. Salt formation at any of $R^3$ through $R^9$, when any of these are basic amine functions, is preferred.

Examples of inorganic and organic acids which may form nontoxic, pharmaceutically acceptable acid addition salts with a compound of formula I are the following: hydrochloric acid, hydrobromic acid , sulfuric acid, phosphoric acid, nitric acid, tartaric acid, citric acid, methanesulfonic acid and the like. Compounds of the formula I usually form acid addition salts with one molar equivalent of the acid.

The compounds of Formula I can be prepared by known methods or obvious modifications thereof. Methods A, B and C described below, are illustrative of the methods for preparing the compounds.

Method A

In Method A, a compound of Formula 1, wherein Z is oxygen and both $R^1$ and $R^2$ and other than hydrogen, is prepared by converting a 6,11-dihydro-5H-pyrido[2,3-b][1,5]benzodiazepin-5-one of the formula

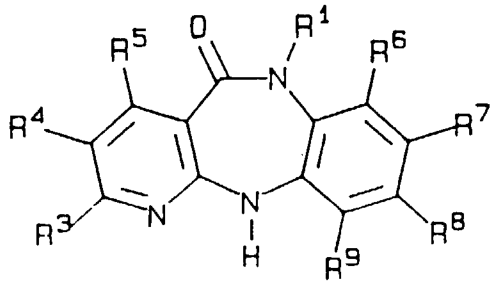

(II)

wherein $R^1$, $R^3$ through $R^9$ have the same meanings set forth with respect to Formula I but $R^1$ is other than H, into the corresponding 11-alkali metal compound, and the alkali metal compound is subsequently reacted with a compound of the formula

$$R^2X \quad \text{(III)}$$

wherein $R^2$ has the same meanings set forth with respect to formula I but is other than hydrogen, and X is a suitable leaving group such as chlorine, bromine or iodine, an appropriate radical of sulfuric acid, an aliphatic or aromatic sulfonic acid ester, or acyloxy.

The reaction is conveniently carried out as a one-batch process, wherein the alkali-metal salts obtained from a compound of formula II are not isolated from the reaction mixture but produced in situ, and, once formed, are reacted further in the same reaction medium.

The conversion of a compound of general formula II into the corresponding alkali metal compound may be effected by reacting a compound of formula II with a lithium alkyl (e.g. n-butyl lithium, or t-butyl lithium), optionally in the presence of tetramethylethylenediamine, a lithium dialkylamide, (e.g. lithium diisopropylamide, lithium dicyclohexylamide and lithium isopropylcyclohexylamide), a lithium aryl (e.g. phenyl lithium), alkali metal hydroxides (e.g. lithium, sodium or potassium hydroxide), alkali metal hydrides (e.g. sodium or potassium hydride) or alkali metal amides (e.g. sodium or potassium amides), or Grignard reagents (e.g. methyl magnesium iodide, ethyl magnesium bromide or phenyl magnesium bromide). The metallation is conveniently carried out in an inert organic solvent at temperatures of between -100°C and the boiling point of the reaction mixture in question. If lithium alkyls, lithium aryls, lithium dialkylamides or Grignard reagents are used for the metallation, the preferred solvents are ethers such as tetrahydrofuran, diethyl ether or dioxan, optionally in a mixture with aliphatic or aromatic hydrocarbons, such as hexane or benzene, and the operation is carried out at temperatures of between -20 and +80°C. When metallation is effected with alkali metal hydrides and alkali metal amides, in addition to the solvents mentioned hereinbefore it is also possible to use xylene, toluene, acetonitrile, dimethylformamide and dimethylsulfoxide, while if alkali metal hydroxides are used it is also possible to use alcohols such as ethanol, methanol and aliphatic ketones such as acetone, as well as mixtures of these solvents with water.

For conversion of the alkali metal substituted 6,11-dihydro-5H-pyrido[2,3-b][1,5]benzodiazepin-5-one thus obtained into a compound of formula I, the solution or suspension of the alkali metal compound is reacted directly, i.e. without isolation of the reaction product with a compound of formula III at room or elevated temperatures, preferably

at the boiling point of the solvent or suspension medium or at the boiling point of the compound III, whichever is lower.

It will be obvious to those skilled in the art that the presence of nucleophilic substituents in the compounds of formula II may require the use of an intermediate of formula II having substituents which are, other than the 11-position nitrogen, not nucleophilic but which can be derivatized to yield the required group. For example, amino or monoalkylamino substituents at any of $R^3$ through $R^9$ are preferably obtained by alkylating or acylating an intermediate of formula II having a nitro group at any of $R^3$ through $R^9$, and subsequently reducing the nitro group, and alkylating, if appropriate, to yield the final product.

Method B

In Method B, a compound of formula I, wherein Z is oxygen and $R^1$ is hydrogen, is obtained by hydrolyzing a compound of formula IV

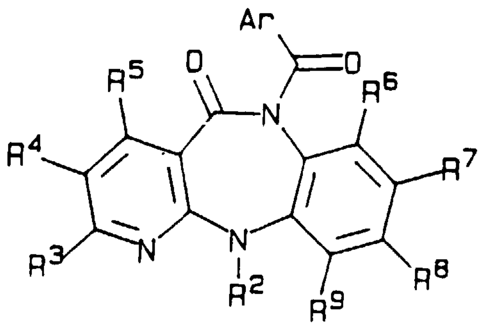

(IV)

wherein the groups $R^2$ through $R^9$ are as hereinbefore defined and Ar is an aromatic or heterocyclic group consisting of one or two nuclei which is or are optionally substituted by halogen, methyl or methoxy, e.g. phenyl, 4-bromophenyl, 1-naphthyl- or 4-pyridinyl.

The hydrolysis is carried out by the action of water or low molecular weight alcohols such as methanol, ethanol, 2-propanol, optionally in the presence of protic or aprotic co-solvents being miscible therein, such as tetrahydrofuran, 1,4-dioxane, dimethylformamide, dimethylacetamide, sulfolane, 1,3-dimethyl-2-imidazolidinone and, where appropriate, in the presence of alkaline or acid catalysts, at temperatures between 0°C and the boiling point of the solvent mixture. As alkaline catalysts, alkali metal hydroxides such as lithium, sodium, calcium and barium hydroxide have proved to be suitable, as acid catalysts, mineral acids, such as aqueous hydrochloric acid, hydrobromic acid, sulfuric acid or also methansulfonic acid or p-toluenesulfonic acid are preferred.

The 6,11-dihydro-5H-pyrido[2,3-b][1,5]benzodiazepin-5-ones of formula II, which are used as starting materials are either specifically described in U.S. patents 3,316,251 or 3,326,900, can be prepared according to general procedures described therein.

Starting materials of formula IV can be prepared by converting a 6-aroyl-6,11-dihydro-5H-pyrido[2,3-b][1,5]benzodiazepin-5-one of the formula

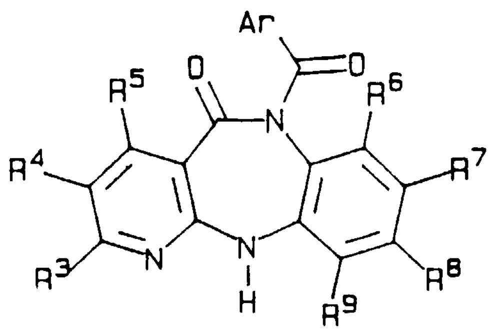

(V)

wherein Ar and $R^3$ through $R^9$ have the same meanings set forth with respect to formula IV, into the corresponding 11-alkali metal compound and reacting the alkali metal compound thus obtained with a compound of formula III.

The conversion of the compound of formula V into the corresponding alkali metal compound may be effected by reacting a compound of formula V with an alkali metal hydride, preferably lithium hydride, sodium hydride or potassium hydride. The reaction is preferably carried out at elevated temperatures and in the presence of an inert organic solvent such as absolute tetrahydrofuran or dimethylformamide.

For conversion of the alkali metal substituted 6-aroyl-6,11-dihydro-5H-pyrido[2,3-b][1,5]benzodiazepin-5-one thus obtained into a compound of formula IV, the solution or suspension of the alkali metal compound is reacted directly, i. e., without isolation, with a compound of formula III at room or elevated temperatures, preferably at the boiling point of the solvent or of compound III.

Starting materials of formula V may be obtained by converting a compound of formula II wherein $R^1$ and $R^2$ are hydrogen atoms to its monosodium compound, preferably by reaction with 1 equivalent of sodium hydride in dimethylformamide, and reacting the alkali metal compound thus formed with an aroyl halide of the formula

$$ArCOHal \qquad (VI)$$

wherein Ar has the same meanings as in compounds of formula IV, and Hal is chlorine, bromine or iodine.

It will be obvious to those skilled in the art that this method is not prefered in those cases wherein any of $R^2$ through $R^9$ are readily hydrolyzable substituents, for example, wherein $R^2$ is alkanoyl or any of $R^3$ through $R^9$ are alkanoylamino or alkoxycarbonyl. In cases wherein $R^2$ is alkanoyl or any of $R^3$ through $R^9$ are alkoxycarbonyl, for example, it is preferable to utilize method A described above; when $R^1$ is hydrogen two equivalents of base must be used. In cases wherein any of $R^3$ through $R^9$ are alkanoylamino, for example, it is preferable to carry out the hydrolysis (and subsequent acylation) on the corresponding nitro derivative, and then reduce the nitro moiety to the amine, followed by acylation to yield the desired product.

Method C

In Method C, a compound of Formula I, wherein Z is sulfur, is obtained by reacting a compound of formula I, wherein Z is oxygen, with a sulfurating agent, such as 2,4-bis(4-methoxyphenyl)-1,3-dithia-2,4-diphosphetane-2,4-disulfide; bis(tricyclohexyltin)sulfide; bis(tri-n-butyltin)sulfide; bis(triphenyltin)sulfide; bis(trimethylsilyl)sulfide or phosphorous pentasulfide. The reaction is carried out in an inert organic solvent such as carbon disulfide, benzene or toluene, at room temperature or higher, preferably an elevated temperature up to the boiling point of the reaction mixture, and preferably under anhydrous conditions. When using the above mentioned tin or silyl sulfide, it is preferable to carry out the sulfurization reaction in the presence of a Lewis acid such as boron trichloride.

It will be obvious to those skilled in the art that the presence of another carbonyl moiety in a compound of formula I, for example, a compound wherein Z is oxygen and any of $R^3$ through $R^9$ is alkanoyl, will require that the ketone carbonyl be protected via known methods by a suitable protecting group prior to the sulfurization reaction; deprotection subsequent to the sulfurization reaction provides the desired compound. Similarly, in cases wherein $R^2$ is, for example, alkanoyl, it will be obvious that the sulfurization reaction should be performed prior to the acylation of the 11-position nitrogen. In those cases wherein the substituents at any of $R^3$ through $R^9$ can be derived from nitro, for example, alkanoylamino, the sulfurization reaction can be performed on the corresponding nitro derivative, followed by an appropriate (known) reduction and finally acylation to yield the desired product.

The above-described compounds of Formula I, and their salts, possess inhibitory activity against HIV-1 reverse transcriptase. When administered in suitable dosage forms, they are useful in the prevention or treatment of AIDS, ARC and related disorders associated with HIV infection. Another aspect of the invention, therefore, is a method for preventing or treating HIV-1 infection which comprises administering to a human being, exposed to or infected by HIV-1, a prophylactically or therapeutically effective amount of a novel compound of Formula I, as described above.

Yet another aspect of the invention comprises a method for preventing or treating HIV-1 infection which comprises administering, to a human being exposed to or infected by HIV-1, a prophylactically or therapeutically effective amount of one of the following known 6,11-dihydro-5H-pyrido[2,3-b][1,5]benzodiazepin-5-ones of the formula

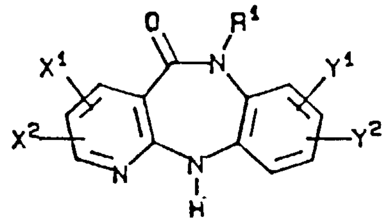

IA

wherein,

$R^1$ is hydrogen, methyl, ethyl or isobutyl; and
$X^1$, $X^2$, $Y^1$ and $Y^2$ are each hydrogen or methyl:

a) 2,4,6,8-tetramethyl-6,11-dihydro-5H-pyrido[2,3-b][1,5]benzodiazepin-5-one;
b) 6-methyl-6,11-dihydro-5H-pyrido[2,3-b][1,5]benzodiazepin-5-one;
c) 6,11-dihydro-5H-pyrido[2,3-b][1,5]benzodiazepin-5-one;
d) 6-ethy-6,11-dihydro-5H-pyrido[2,3-b][1,5]benzodiazepin-5-one;
e) 6,8,9-trimethyl-6,11-dihydro-5H-pyrido[2,3-b][1,5]benzodiazepin-5-one;
f) 6-ethyl-8,9-dimethyl-6,11-dihydro-5H-pyrido[2,3-b][1,5]benzodiazepin-5-one;
g) 6-isobutyl-6,11-dihydro-5H-pyrido[2,3-b][1,5]benzodiazepin-5-one; and,
Ii) 6-ethyl-9-methyl-6,11-dihydro-5H-pyrido[2,3-b][1,5]benzodiazepin-5-one.

The above described compounds of formula 1A also inhibit HIV-1 reverse transcriptase (HIV-1 RT).

Said compounds are known from US patents Nos. 3,316,251; 3,326.900 and 4,167,570.

A further aspect of the invention comprises the novel 5-thione analogues of the above-mentioned known compounds of formula IA. These 5-thione analogues can be prepared from the known 5-ones using the sulfurization reaction described in Method C, above. These analogues also inhibit HIV-1 RT and the invention further comprises a method for preventing or treating HIV-1 infection which comprises administering, to a human being exposed to or infected by HIV-1, a prophylactically or therapeutically effective amount of one or more of these 5-thione analogues.

The above described compounds of formulas I and IA, and the 5-thione analogues of the compounds of formula IA may be administered in single or divided doses by the oral, parenteral or topical routes. A suitable oral dosage for such compounds would be in the range of about 10 to 500 mg per day. In parenteral formulations, a suitable dosage unit may contain from about 1 to 50 mg of said compounds, whereas for topical administration, formulations containing about 0.01 to 1% active ingredient are preferred. It should be understood, however, that the dosage administration from patient to patient will vary and the dosage for any particular patient will depend upon the clinician's judgement, who will use as criteria for fixing a proper dosage the size and condition of the patient as well as the patient's response to the drug.

When the compounds of formulas I, IA, or the 5-thione analogues of the compounds of formula IA are to be administered by the oral route, they may be administered as medicaments in the form of pharmaceutical preparations which contain them in association with a compatible pharmaceutical carrier material. Such carrier material can be an inert organic or inorganic carrier material suitable for oral administration. Examples of such carrier materials are water, gelatin, talc, starch, magnesium stearate, gum arabic, vegetable oils, polyalkylene-glycols, petroleum jelly and the like. The pharmaceutical preparations can be prepared in a conventional manner and finished dosage forms can be solid dosage forms, for example, tablets, dragees, capsules, and the like, or liquid dosage forms, for example, solutions, suspensions, emulsions and the like. The pharmaceutical preparations may be subjected to conventional pharmaceutical operations such as sterilization. Further, the pharmaceutical preparations may contain conventional adjuvants such as preservatives, stabilizers, emulsifiers, flavor-improvers, wetting agents, buffers, salts for varying the osmotic pressure and the like. Solid carrier material which can be used include, for example, starch, lactose, mannitol, methyl cellulose, microcrystalline cellulose, talc, silica, dibasic calcium phosphate, and high molecular weight polymers (such as polyethylene glycol).

For parenteral use, a compound of formula I or IA, or a 5-thione analogue of a compound of formula IA can be administered in an aqueous or non-aqueous solution, suspension or emulsion in a pharmaceutically acceptable oil or a mixture of liquids, which may contain bacteriostatic agents, antioxidants, preservatives, buffers or other solutes to render the solution isotonic with the blood, thickening agents, suspending agents or other pharmaceutically acceptable additives. Additives of this type include, for example, tartrate, citrate and acetate buffers, ethanol, propylene glycol, polyethylene glycol, complex formers (such as EDTA), antioxidants (such as sodium bisulfite, sodium metabisulfite,

and ascorbic acid), high molecular weight polymers (such as liquid polyethylene oxides) for viscosity regulation and polyethylene derivatives of sorbitol anhydrides. Preservatives may also be added if necessary, such as benzoic acid, methyl or propyl paraben, benzalkonium chloride and other quaternary ammonium compounds.

The compounds of formulas I and IA and the 5-thione analogues of the compounds IA may also be administered as solutions for nasal application and may contain in addition to the compounds of this invention suitable buffers, tonicity adjusters, microbial preservatives, antioxidants and viscosity-increasing agents in an aqueous vehicle. Examples of agents used to increase viscosity are polyvinyl alcohol, cellulose derivatives, polyvinylpyrrolidone, polysorbates or glycerin. Microbial preservatives added may include benzalkonium chloride, thimerosal, chlorobutanol or phenylethyl alcohol.

Additionally, the compounds of formulas I and IA and the 5-thiones analogues of the compounds of formula IA can be administered by suppository.

As stated before, the above described compounds of formulas I and IA and the 5-thione analogues of the compounds of formula IA inhibit the enzymatic activity of HIV-1 RT. Based upon testing of these compounds, as described below, it is known that they inhibit the RNA-dependent DNA polymerase activity of HIV-1 RT. Based upon other testing, not described herein, it is believed that they also inhibit the DNA polymerase activity of HIV-1 RT.

Utilizing the Reverse Transcriptase (RT) Assay descibed below, compounds can be tested for their ability to inhibit the RNA-dependent DNA polymerase activity of HIV-1 RT. Certain specific compounds of formula I described in the Examples which appear below, were so tested. The results of this testing appear in Table I, below. The above-described compounds of formula IA were also so tested. The results of this testing appear in Table II, below.

## REVERSE TRANSCRIPTASE (RT) ASSAY

Assay Theory:

Among the enzymes for which Human Immunodeficiency Virus (HIV-1) encodes is a reverse transcriptase (1), so-named because it transcribes a DNA copy from an RNA template. This activity can be quantitatively measured in a cell-free enzyme assay which has been previously described (2), and is based upon the observation that reverse transcriptase is able to use a synthetic template [poly r(C) primed with oligo d(G)] to transcribe a radio-labelled, acid-precipitable DNA strand utilizing $^3$H-dGTP as a substrate.

Materials

a) Preparation of the enzyme

Reverse transcriptase enzyme from the LAV strain of Human Immunodeficiency Virus (HIV-1) (1) was isolated from the bacterial strain JM109 (3) expressing the DNA clone pBRTprt1+ (2) which is under the control of the lac promoter in the expression vector pIBI21 (4). An overnight culture grown in 2XYT medium (37°C, 225 rpm) (5) supplemented with 100 µg/ml ampicillin for positive selection is inoculated at a 1:40 dilution into M9 medium supplemented with 10µg/ml thiamine, 0.5% casamino acids, and 50 µg/ml ampicillin (5). The culture is incubated (37°C, 225 rpm) until it reaches an OD540 of 0.3-0.4. At that time the repressor inhibitor IPTG (isopropyl b-D-thiogalactopyranoside) is added to 0.5mM and the mixture is incubated for 2 additional hours. Bacteria are pelleted, resuspended in a 50mM Tris, 0.6mM EDTA, 0.375M NaCl buffer and digested by the addition of lysozyme (1mg/ml) for 30 minutes on ice. The cells are lysed by the addition of 0.2% NP-40 and brought to 1M NaCl.

After removal of the insoluble debris by centrifugation, the protein is precipitated by the addition of 3 volumes of water-saturated ammonium sulfate. The enzyme is pelleted, resuspended in RT buffer (50mM Tris pH 7.5. 1mM EDTA, 5mM DTT, 0.1% NP-40, 0.1M NaCl, and 50% glycerol), and stored at -70°C for further use.

b) Composition of 2X concentrated stock reaction mixture

| Stock Reagent | 2X Mix Concentrtation |
|---|---|
| 1M Tris pH 7.4 | 100mM |
| 1M Dithiothreitol | 40mM |
| 1M NaCl | 120mM |
| 1% Nonidet P-40 | 0.1% |
| 1M MgCl | 4mM |

(continued)

| Stock Reagent | 2X Mix Concentrtation |
|---|---|
| [poly r(C)/oligo d(G)](5:1) | 2μg/ml |
| $^3$H-dGTP (81μM) | 0.6μM |

Assay Procedure:

The 2X concentrated stock reaction mixture is aliquoted and stored at -20°C. The mixture is stable and thawed for use in each assay. This enzyme assay has been adapted to a 96 well microtiter plate system, and has been previously described (6). Tris buffer (50 mM, pH 7.4), vehicle (solvent diluted to match the compound dilution), or compounds in vehicle are dispensed into 96-well microtiter plates (10μl/well: 3 wells/compound). The HIV-1 RT enzyme is thawed and diluted in 50mM Tris pH 7.4 so that fifteen μl of diluted enzyme contain 0.001 Unit (one unit is that amount of enzyme to transform 1 micromole of substrate per minute at 25°C) and 15μl are dispensed per well. Twenty μl of 0.12 - 0.5M EDTA are added to the first three wells of the microtiter plate. EDTA chelates the $Mg^{++}$ present and prevents reverse transcription. This group serves as background polymerization which is subtracted from all other groups. Twenty-five ul of the 2X reaction mixture are added to all wells and the assay is allowed to incubate at room temperature for 60 minutes. The assay is terminated by precipitating the DNA in each well with 50μl of trichloracetic acid (TCA) (10% w/v) in sodium pyrophosphate (1% w/v). The microtiter plate is incubated for 15 minutes at 4°C and the precipitate is fixed onto #30 glass fiber paper (Schleicher & Schuell) using a Skatron semi-automatic harvester. The filters are then washed with additional TCA (5%) containing sodium pyrophosphate (1%), rinsed with aqueous ethanol (70%), dried, and transferred to scintillation vials (6). Each vial receives 2 mls of scintillation cocktail and is counted in a Beckman beta counter.

The calculation for percent inhibition is as follows:

$$\% \text{ inhibition} = \frac{\text{CPM Mean Test Value - CPM Mean Control Value X100}}{\text{CPM Mean Control Value}}$$

References:

1. Benn, S., et al., SCIENCE 230:949, 1985
2. Farmerie, W.G. et. al., SCIENCE 236:305, 1987
3. Yanisch-Perron, C., Viera, J., and Messing, J., GENE 33:103, 1985
4. International Biotechnologies, Inc., P.O. Box 9558, New Haven, CT 06535
5. Maniatis, T, Fritsch, E.F., and J. Sambrook, eds. MOLECULAR CLONING: A LABORATORY MANUAL, Cold Spring Harbor Laboratory, 1982
6. Spira, T., et. al., J. Clinical Microbiology, 25:97, 1987.

In order to confirm that compounds which are active in the RT Assay also have the ability to inhibit HIV-1 replication in a living system, several compounds of formula I were also tested in the Human T-Cell Culture Assay described below. The results of this testing appear in Table I.

<u>HUMAN T CELL CULTURE ASSAY</u>

Assay Theory:

Formation of syncytia is a feature of <u>in vitro</u> cultures of CD4+ T-cells infected with HIV-1. In this assay, T-cells are treated with a putative replication inhibiting compound and then infected with HIV-1. After incubation the culture is checked for the formation of syncytia. The absence or reduction is the number of syncytia is used as a measure of the test compound's ability to inhibit HIV replication.

Assay Method:

The target cells, designated c8166, are a subclone of human lymphoma cells of T-cell origin and are established at an initial density of $5x10^4$ per 100 ul in RPMI 1640 (+ 10% fetal bovine serum) culture medium in 96 well flat bottom plates. A selected amount of test compound, dissolved in DMSO is included. After 24 hours, 50-100 $TCID_{50's}$ (the dose that results in induced effect in 50% of test cultures) of the HTLV-IIIB strain of HIV-1 (2) are innoculated into each

culture. Control cultures receive compound or virus only. Four days after virus challenge, cultures are visually examined for the frequency and distribution of virus-induced giant cell syncytia. The percent inhibition by the test compound is determined by comparison with control values. Confirmation of the presence or absence of virus replication is accomplished by harvesting the cell free culture fluids from all experimental groups to determine the present or absence of infectious progeny through the induction of syncytia formation in secondary human T-cell cultures after 3 days.

REFERENCES:

(1) M. Somasundaran and H.L. Robinson, Science 242, 1554 (1998)
(2) G.M. Shaw, R.H. Hahn, S.K. Arya, J.E. Groopman, R.C. Gallo and F. Wong-Staal, Science 226, 1165 (1984)

In order to assess the specificity of the enzyme inhibitory activity of the above described compounds, a few were tested, using known per se assay methods, for their ability to inhibit Feline Leukemia Virus-derived reverse transcriptase and Galf Thymus-derived DNA alpha-polymerase. None of the compounds so tested was observed to possess significant inhibitory activity against these enzymes. These results indicate that the enzyme inhibitory activity of the above described compounds is directed rather specifically against HIV-1 RT.

In order to roughly assess the cytotoxicity of the compounds of formula 1, several such compounds were tested in the MTT Cellular Cytotoxicity Assay described below. The results of this testing are reported in Table I, below. Compounds having a relatively high $EC_{50}$ are preferred.

MTT ASSAY FOR CELLULAR CYTOTOXICITY

Assay Theory:

The MTT [3-(4,5-dimethylthiazol-2yl)-2,5-diphenyl tetrazolium bromide) assay is based on cleavage of tetrazolium bromide by metabolically active cells, resulting in a highly quantitative blue color. This assay has been previously described (1) but has been optimized for the purposes of the testing reported herein.

Assay Method:

The H9 cell line (2), an established human lymphoma suspension cell line grown in RPMI 1640 supplemented with 10% fetal bovine serum is used as the target cell line in the assay. Cells (100µl) are plated in microtest plate wells at a concentration of $10^5$ cells per ml in the presence of varying concentrations of inhibitor. The cells are incubated at 37°C in a humidified $CO_2$ incubator. Five days later 20µl of MTT (5mg/ml in RPMI 1640, sonicated, 0.2 micron filtered, and stored at 4°C) is added to each well. After 4 hours additional incubation at 37°C, 60µl of Triton-X is added to each well and thoroughly mixed to aid the solubilization of the crystals. Absolute ethanol (5µl) is added to each well and the resulting mixture is incubated for 30 minutes at 60°C and immediately read on a plate reader (Dynatech) at a wavelength of 570nm.

Data from this assay are used to generate a nonlinear regression analysis, which yields the $EC_{50}$.

References:

1. Mosmann, Tim, J. Immunol. Methods, 65:55, 1983.
2. Jacobs, J.P., J. Natl. Cancer Inst., 34:231, 1965.

TABLE I

| Compound of Example No. | RT Inhibition % @ 10µg/ml | T-Cell Culture Assay (%inhibition) | Cytotoxity Assay ($EC_{50}$) |
|---|---|---|---|
| 1 | 37 | NT | NT |
| 2 | 97 | 100%@ 4µM | 80µM |
| 3 | 92 | NT | NT |
| 4 | 79 | " | 49µM |
| 5 | 94 | 100%@19µM | 50µM |
| 6 | 46 | NT | NT |
| 7 | 50 | " | " |

TABLE I (continued)

| Compound of Example No. | RT Inhibition % @ 10µg/ml | T-Cell Culture Assay (%inhibition) | Cytotoxity Assay ($EC_{50}$) |
|---|---|---|---|
| 8 | 55 | " | 37µM |
| 9 | 92 | " | 4µM |
| 10 | 68 | " | NT |
| 11 | 88 | " | " |
| 12 | 89 | " | " |
| 13 | 91 | " | " |
| 14 | 75 | " | " |
| 15 | 39 | " | 18 |
| 16 | 44 | " | NT |
| 17 | 49 | " | 9 |
| 18 | 29 | " | NT |
| 19 | 90 | " | 85 |
| 20 | 69 | " | NT |
| 21 | 96 | " | " |
| 22 | 100 | 100%@3µM | " |
| 23 | 100 | NT | " |
| 24 | 100 | " | " |
| 25 | 73 | " | " |
| 26 | 81 | " | " |
| 27 | 64 | " | " |
| 28 | 65 | " | " |
| 29 | 99 | " | " |
| 30 | 94 | " | " |
| 31 | 92 | " | " |
| 32 | 51 | " | " |
| 33 | 97 | " | " |
| 34 | 85 | " | " |
| Note: NT = not tested | | | |

TABLE II

| Compound of Formula IA | RT Inhibition % @ 10µg/ml |
|---|---|
| a | 58 |
| b | 43 |
| c | 22 |
| d | 69 |
| e | 65 |
| f | 95 |
| g | 33 |
| h | 78 |

The following examples further illustrate the present invention and will enable others skilled in the art to understand the invention more completely. It should be understood, however, that the invention is not limited to the particulars given in the examples.

Example 1

6,11-Dihydro-11-methyl-5H-pyrido[2,3-b][1,5]benzodiazepin-5-one

a) 6-Benzoyl-6,11-dihydro-5H-pyrido[2,3-b][1,5]benzodiazepin-5-one

A suspension of 14.8 g (0.07 mol) of 6,11-dihydro-5H-pyrido[2,3-b]1,5]benzodiazepin-5-one in 200 ml dimethylformamide was heated to 120°C, cooled down to 80° after complete dissolution, whereupon 2.2g (0.074 mol) of an 80% dispersion of sodium hydride in mineral oil were added. Thereafter, the mixture was stirred at 60°C for 45 minutes, 8.6 ml (0.075 mol) of benzoyl chloride were dropped in and the reaction mixture was stirred at the same temperature for a further 15 minutes. The mixture was cooled to room temperature and after being stirred for two hours was set aside overnight. The resulting precipitate, mostly consisting of starting material, was filtered off. The filtrate was then evaporated in vacuo, the residue was admixed with 100 ml of water and 100 ml of dichloromethane and filtered again. The organic phase was separated and evaporated in vacuo. The oily residue was recrystallized from chloroform/ethylacetate 1/1 (v/v) yielding slightly yellowish crystals of m.p. 198-201°C which were identified as 6-benzoyl-6,11-dihydro-5H-pyrido[2,3-b][1,5]benzodiazepin-5-one. The yield was 3.5g (16% of theory).

b) 6-Benzoyl-6,11-dihydro-11-methyl-5H-pyrido[2,3-b][1,5]benzodiazepin-5-one

12.67 g (0.06 mol) of the product obtained in step a) were dissolved in 190 ml of absolute dimethylformamide and the resulting solution was admixed with 1.95 g (0.065 mol) of an 80% dispersion of sodium hydride in mineral oil, whereupon hydrogen evolved and the mixture became reddish and slightly warm. The mixture was stirred at room temperature for 15 minutes, then cooled to +10°C and admixed with 4.38 ml (0.07 mol) of methyl iodide and subsequently was stirred at room temperature for a further 45 minutes. The solvent was removed by distillation in vacuo. The residue was washed with water, dissolved in hot ethanol and the resulting solution was treated with charcoal and filtered while hot. The filtrate was set aside at room temperature for several hours, and the resulting precipitate was filtered by suction. The product had a m.p. of 181-183°C and was identified as 6-benzoyl-6,11-dihydro-11-methyl-5H-pyrido-[2,3-b][1,5]benzodiazepin-5-one. The yield was 12.54 g (63% of theory).

c) 6,11-Dihydro-11-methyl-5H-pyrido[2,3-b][1,5]benzodiazepin-5-one

A mixture of 9.4 g (0.0285 mol) of the product obtained in step b, 150 ml ethanol and 5 ml concentrated aqueous hydrochloric acid was refluxed for five hours while stirring. The clear yellowish solution that had been formed was set aside overnight. The resulting precipitate was collected by filtration and thoroughly washed with diluted aqueous ammonia. The product was dried overnight in a stream of nitrogen at room temperature and thereafter was recrystallized from n-propanol. The colorless product had a m.p. of 235-237°C and was identified as 6,11-dihydro-11-methyl-5H-pyrido-[2,3-b]-[1,5]benzodiazepin-5-one. The yield was 3.3 g (51% of theory).

Example 2

6,11-Dihydro-11-ethyl-6-methyl-5H-pyrido[2,3-b][1,5]benzodiazepin-5-one

Under nitrogen cover, 7.90 g (0.035 mol) of 6,11-dihydro-6-methyl-5H-pyrido[2,3-b][1,5]benzodiazepin-5-one were suspended in 79 ml of anhydrous dimethylformamide. To this suspension 1.25 g (0.052 mol) of sodium hydride were added in small portions within 10 minutes. The temperature arose to 60°C with development of hydrogen. Stirring was continued for a further 45 minutes at the same temperature. Afterwards, the batch was left to cool to ambient temperature. Ethyl iodide (3.38 ml; 6.534 g, 0.0419 mol) was dropped in within about 10 minutes. The batch was stirred for further 15 minutes at ambient temperature. After this time no starting material was found in the reaction mixture by thin-layer-chromatography.

In order to destroy still present sodium hydride, 1 ml of methanol was added. The reaction mixture was evaporated in vacuo, and the residue was distributed between 100 ml of chloroform and 100 ml of water, the red organic phase was dried over sodium sulfate, 1 g of charcoal was stirred in, the batch was filtered and the filtrate was evaporated to dryness in vacuo. The high viscous red oil obtained was purified by column chromatography using 300 g of silica gel (0.2-0.5 mm) and as eluent chloroform/ethyl acetate 1/1 (v/v). By evaporation of the fraction containing the compound, 7.0 g of a slightly red colored product were obtained, which was recrystallized with cyclohexane, yielding 5 g (56% of theory) of 6,11-Dihydro-11-ethyl-6-methyl-5H- pyrido[2,3-b][1,5]benzodiazepin-5-one as colorless crystals of m.p. 106-111°C.

Example 3

6,11-Dihydro-6-methyl-11-propyl-5H-pyrido[2,3-b][1,5]benzodiazepin-5-one

Using a procedure analogous to that described in Example 2 6,11-dihydro-6-methyl-11-propyl-5H-pyrido[2,3-b][1,5]benzodiazepin-5-one, m.p. 96-98°C (recrystallized from petroleum ether) was prepared from 6,11-dihydro-6-methyl-5H-pyrido[2,3-b]-[1,5]benzodiazepin-5-one and 1-iodopropane. The yield was 36% of theory.

Example 4

6,11-Dihydro-6,11-dimethyl-9-(trifluoromethyl)-5H-pyrido[2,3-b][1,5]benzodiazepin-5-one

a) 6,11-Dihydro-6-methyl-9-(trifluoromethyl)-5H-pyrido[2,3-b][1,5]benzo-diazepin-5-one

13.26 g (0.475 mol) of 6,11-dihydro-9-(trifluoromethyl)-5H-pyrido[2,3-b][1,5]benzodiazepin-5-one were dissolved in a mixture consisting of 40 ml of a potassium tert. butoxide solution (from 2.14 g of potassium and 40 ml of tert. butanol) and 100 ml of absolute dioxane, and the resulting solution was refluxed for two hours. Thereafter, 14.2 g (0.1 mol) of methyliodide were added and the mixture was refluxed for four hours. The reaction mixture was then filtered to remove the precipitated potassium iodide, and the filtrate was evaporated in vacuo. The residue was recrystallized from ethanol, yielding a compound having a melting point of 169-172°C which was identified to be 6,11-dihydro-6-methyl-9-(trifluoromethyl)-5H-pyrido[2,3-b][1,5]benzodiazepin-5-one. The yield was 26% of theory.

b) 6,11-Dihydro-6,11-dimethyl-9-(trifluoromethyl)-5H-pyrido[2,3-b][1,5]benzodiazepin-5-one

Using a procedure analogous so that described in Example 2, 6,11-dihydro-6,11-dimethyl-9-(trifluoromethyl)-5H-pyrido-[2,3-b][1,5]-benzodiazepin-5-one, m.p. 142-145°C (recrystallized from ethanol) was prepared from the product of step a) and methyl iodide. The yield was 48% of theory.

Example 5

6,11-Dihydro-6-methyl-11-methylethyl-5H-pyrido[2,3-b][1,5]benzodiazepin-5-one

Using a procedure analogous to that described in Example 2, 6,11-Dihydro-6-methyl-11-isopropyl-5H-pyrido[2,3-b][1,5]benzodiazepin-5-one, m.p. 144-147°C (recrystallized from cyclohexane) was prepared from 6,11-Dihydro-6-methyl-5H-pyrido[2,3-b][1,5]benzodiazepin-5-one and 2-bromopropane. The yield was 17% of theory.

Example 6

11-Butyl-6,11-dihydro-6-methyl-5H-pyrido[2,3-b][1,5]-benzodiazepin-5-one

Using a procedure analogous to that described in Example 2, 11-butyl-6,11-dihydro-6-methyl-5H-pyrido[2,3-b][1,5]benzodiazepin-5-one, m.p. 56-58°C (recrystallized from petroleum ether) was prepared from 6,11-dihydro-6-methyl-5H-pyrido[2,3-b[1,5]benzodiazepin-5-one, and 1-iodobutane. The yield was 40% of theory.

Example 7

6,11-Dihydro-11-(2-hydroxyethyl)-6-methyl-5H-pyrido[2,3-b][1,5]benzodiazepin-5-one

Using a procedure analogous to that described in Example 2, 22.52g (0.1 mol) of 6,11-dihydro-6-methyl-5H-pyrido[2,3-b][1,5]benzodiazepin-5-one were converted to 6,11-dihydro-6-methyl-11-[2-[(2-tetrahydropyranyl)oxy]ethyl]-5H-pyrido-[2,3-b][1,5]benzodiazepin-5-one by reaction with 21.8g (0.132 mol) of 1-chloro-2-[(2-tetrahydropyranyl)-oxy]-ethane. The raw material thus obtained was dissolved in a mixture of 500 ml ethanol and 100 ml of concentrated aqueous hydrochloric acid. After having been refluxed for two hours, the reaction mixture was evaporated in vacuo. The residue was triturated with ethanol, the precipitated crystals were filtered off and the solvent was removed from the filtrate by distillation in vacuo. The residue was purified by column chromatography on silica gel (0.2-0.5 mm) using chloroform/ethylacetate/methanol 5/5/1 (v/v/v) as an eluent. Colorless crystals of m.p. 133-134°C (after recrystallization from xylene) were obtained in a yield of 9.0 g (33% of theory).

Example 8

8-Chloro-6,11-dihydro-6,11-dimethyl-5H-pyrido[2,3-b][1,5]benzodiazepin-5-one

Using a procedure analogous to that described in Example 2, 8-chloro-6,11-dihydro-6,11-dimethyl-5H-pyrido[2,3-b][1,5]benzodiazepin-5-one, m.p. 188-189°C (recrystallized from ethanol) was prepared from 8-chloro-6,11-dihydro-6-methyl-5H-pyrido[2,3-b][1,5]benzodiazepin-5-one and methyl iodide. The yield was 33% theory.

Example 9

6,11-Dihydro-6,8,9,11-tetramethyl-5H-pyrido[2,3-b][1,5]-benzodiazepin-5-one

Using a procedure analogous to that described in Example 2, 6,11-dihydro-6,8,9,11-tetramethyl-5H-pyrido[2,3-b][1,5]benzodiazepin5-one, m.p. 189-192°C (recrystallized twice from ethanol) was prepared from 6,11-dihydro-6,8,9-trimethyl-5H-pyrido 2,3-b][1,5]benzodiazepin-5-one and methyl iodide. The yield was 47% of theory.

Example 10

6,11-Dihydro-6,11-dimethyl-5H-pyrido[2,3-b][1,5]benzodiazepin-5-one

Using a procedure analogous to that described in Example 2, 6,11-dihydro-6,11-dimethyl-5H-pyrido[2,3-b][1,5]benzodiazepin-5-one, m.p. 126-128°C (recrystallized from cyclohexane) was prepared from 6,11-dihydro-6-methyl-5H-pyrido[2,3-b][1,5]benzodiazepin-5-one and methyl iodide. The yield was 53% of theory.

Example 11

6,11-Dihydro-6-ethyl-11-methyl-5H-pyrido[2,3-b][1,5]benzodiazepin-5-one

Using a procedure analogous to that described in Example 2, 6,11-dihydro-6-ethyl-11-methyl-5H-pyrido[2,3-b][1,5]-benzodiazepin-5-one, m.p. 118-119°C (recrystallized twice from cyclohexane) was prepared from 6,11-dihydro-6-ethyl-5H-pyrido- [2,3-b][1,5]benzodiazepin-5-one and methyl iodide. The yield was 53% of theory.

Example 12

6,11-Dihydro-11-methyl-6-propyl-5H-pyrido[2,3-b][1,5]benzodiazepin-5-one

Using a procedure analogous to that described in Example 2, 6,11-dihydro-11-methyl-6-propyl-5H-pyrido[2,3-b][1,5]benzodiazepin-5-one, m.p. 95.5-97.5°C (recrystallized from petroleum ether) was prepared from 6,11-dihydro-6-propyl-5H-pyrido[2,3-b][1,5]benzodiazepin-5-one and methyl iodide. The yield was 71% of theory.

Example 13

6,11-Dihydro-11-methyl-6-methylethyl-5H-pyrido[2,3-b][1,5]benzodiazepin-5-one

Using a procedure analogous to that described in Example 2, 6,11-dihydro-11-methyl-6-isopropyl-5H-pyrido[2,3-b][1,5]-benzodiazepin-5-one, m.p. 98-100°C (recrystallized from petroleum ether) was prepared from 6,11-dihydro-6-5H-pyrido[2,3-b][1,5]-benzodiazepin-5-one and methyl iodide. The yield was 61% of theory.

Example 14

6-Butyl-6,11-dihydro-11-methyl-5H-pyrido[2,3-b][1,5] benzodiazepin-5-one

Using a procedure analogous to that described in Example 2, 6-butyl-6,11-dihydro-11-methyl-5H-pyrido[2,3-b][1,5]benzodiazepin-5-one, a colorless, viscous liquid of b.p. 150-152°C (0.03 mm Hg) was prepared from 6-butyl-6,11-dihydro-5H-pyrido[2,3-b][1,5]-benzodiazepin-5-one and methyl iodide. The yield was 57% of theory.

Example 15

6,11-Dihydro-2,6,8,9,11-pentamethyl-5H-pyrido[2,3-b][1,5]benzodiazepin-5-one

Using a procedure analogous to that described in Example 2, 6,11-dihydro-2,6,8,9,11-pentamethyl-5H-pyrido [2,3-b][1,5]benzodiazepin-5-one, m.p. 143-145°C (recrystallized from methanol) was prepared from 6,11-dihydro-2,6,8,9-tetramethyl-5H-pyrido[2,3-b][1,5]benzodiazepin-5-one and methyl iodide. The yield was 13% of theory.

Example 16

6,11-Dihydro-2,4,6,8,9,11-hexamethyl-5H-pyrido[2,3-b][1,5]benzodiazepin-5-one

Using a procedure analogous to that described in Example 2, 6,11-dihydro-2,4,6,8,9,11-hexamethyl-5H-pyrido [2,3-b][1,5]-benzodiazepin-5-one, m.p. 148-151°C (recrystallized from ligroin, b.p. 100-140°C) was prepared from 6,11-Dihydro-2,4,6,8,9-pentamethyl-5H-pyrido-[2,3-b][1,5]benzodiazepin-5-one and methyl iodide. The yield was 44% of theory.

Example 17

Mixture of 6,11-Dihydro-2,4,6,8,11-and 6,11-dihydro-2,4,6,9,11- pentamethyl-5H-pyrido[2,3-b][1,5]benzodiazepin-5-one

Using a procedure analogous to that described in Example 2, a mixture of 6,11-dihydro-2,4,6,8,11- and 6,11-dihydro-2,4,6,9,11-pentamethyl-5H- pyrido[2,3-b][1,5]benzodiazepin-5-one, m.p. 174-176°C (recrystallized from ligroin, b. p. 100-140°C) was prepared from a mixture of 6,11-dihydro-2,4,6,8 and 6,11-dihydro-2,4,6,9-tetramethyl- 5H-pyrido [2,3-b][1,5]benzodiazepin-5-one and methyl iodide. The yield was 12% of theory.

Example 18

11-Acetyl-6,11-dihydro-6-methyl-5H-pyrido[2,3-b][1,5]benzodiazepin-5-one

5.0 g (0.0222 mol) of 6,11-dihydro-6-methyl-5H-pyrido-[2,3-b][1,5]-benzodiazepin-5-one were suspended in a mixture consisting of 2.0 g (0.0255 mol) of acetyl chloride, 10 ml benzene and 2.2g (0.022 mol) of triethylamine and the resulting suspension was refluxed for 7 hours while stirred. The reaction mixture was filtered while hot, and the filtrate was evaporated in vacuo. The residue was twice recrystallized from cyclohexane using animal charcoal as an absorbent, yielding a colorless compound having a melting point of 140-142°C, which was identified as 11-acetyl-6,11-dihydro-6-methyl-5H-pyrido[2,3-b][1,5]benzodiazepin-5-one. The yield was 59% of theory.

Example 19

11-Acetyl-6,11-dihydro-6,8,9-trimethyl-5H-pyrido[2,3-b][1,5]benzodiazepin-5-one

Using a procedure analogous to that described in Example 20, 11-acetyl-6,11-dihydro-6,8,9-trimethyl-5H-pyrido [2,3-b][1,5]benzodiazepin-5-one, m.p. 168-170°C (recrystallized from cyclohexane) was prepared from 6,11-dihydro-6,8,9-trimethyl-5H-pyrido[2,3-b][1,5]benzodiazepin-5-one and acetyl chloride. The yield was 43% of theory.

Example 20

6,11-bis-(methylthiomethyl)-9-chloro-6,11-dihydro-5H-pyrido-[2,3-b][1,5]benzodiazepin-5-one

Using a procedure analogous to that described in Example 2, 6,11-bis-(methylthiomethyl)-9-chloro-6,11-dihydro-5H-pyrido-[2,3-b][1,5]benzodiazepin-5-one, m.p. 181-182°C (recrystallized from 1,2-dichloroethane) was prepared from 9-chloro-6,11-dihydro-5H-pyrido[2,3-b][1,5]benzodiazepin-5-one and 2 equivalents of methylthiomethyl chloride. The yield was 31% of theory.

Example 21

6,11-Diethyl-6,11-dihydro-5H-pyrido[2,3-b][1,5]benzodiazepin-5-one

11H-Pyrido[2,3-b][1,5]benzodiazepin-5-one 46g of o-phenylenediamine (0,43 mol) and 46g of 2-chloronicotinic acid (0.29 mol) were mixed and heated to between 110 and 120°C. The mixture melted and an exothermic reaction ensued. The reaction mixture was thereafter heated for two hours at 120°C, cooled and then washed with ethanol. The crude product was crystallized from acetic acid to yield 11H-pyrido[2,3-b][1,5] benzodiazepin-5-one as a yellow solid (449), m.p. 280-285°C.

5g of the compound prepared above (0.024 mol) and 1.2g of sodium hydride (0.05 mol) were heated in dry dimethylformamide (100ml) for two hours. Ethyl iodide ((7.48g; 0.048 mole) was then added dropwise over 10 minutes while maintaining the temperature at 35-40°C. The reaction mixture was stirred overnight at room temperature and then poured into cold water. The resulting mixture was extracted with ether, the ether extract dried over anhydrous sodium sulfate, filtered and evaporated to dryness. The resulting oil was purified through silica gel chromatography, and crystallized from n-heptane to yield the title compound (2.3g), m.p. 108-110°C.

Example 22

6,11-Dihydro-11-ethyl-6-methyl-5H-pyrido[2,3-b][1,5]benzodiazepin-5-thione

A mixture of 4.00 (0.016 mol) of 6,11-dihydro-11-ethyl-6-methyl-5H-pyrido[2,3-b][1,5]benzodiazepin-5-one, prepared as in Example 2, and 3.2g (0.008 mol) of Lawesson's reagent [2,4-bis(4-methoxyphenyl)-1,3-dithia-2,4-diphosphetane-2,4-disulfide] in 50 ml of toluene was refluxed for 2 1/2 hr. The solvent was then removed in vacuo and the residue purified on a silica gel column using methylene chloride. Removal of the solvent in vacuo gave 3.2g (75%) of yellow oil. The oil was dissolved in cyclohexane and after standing at room temperature a yellow solid resulted. Recrystallization from cyclohexane gave 2.1g (49% theory), of yellow needles, m.p. 103-106°C, which was identified to be 5,11-dihydro-11-ethyl-6-methyl-5H-pyrido[3,2-b][1,5]diazepin-5-thione.

Example 23

6,11-Dihydro-11-ethyl-5H-pyrido[2,3-b][1,5]benzodiazepin-5-one

A mixture of 15g of 6,11-dihydro-5H-pyrido[2,3-b][1,5]benzodiazepin-5-one and 150 ml DMF was heated to 50°C. To the slurry was added 7.5 g (2 equiv) of 50% sodium hydride in mineral oil dispersion, during which time the temperature rose to 65°C. The mixture was cooled to 40°C and then 6.0 ml of iodoethane was added. The resulting mixture was stirred at room temperature overnight, then poured into 700 ml of water and extracted with 700 ml of ether. The other phase was dried ($MgSO_4$) and concentrated to dryness. The crude product was purified by column chromatography on basic alumina to yield 3.6g (21% of theory) of 6,11-dihydro-11-ethyl-5-pyrido[2,3-b][1,5]benzodiazepin-5-one, m.p. 210-220°C.

Example 24

6,11-Dihydro-11-ethyl-5H-pyrido[2,3-b][1,5]benzodiazepin-5-thione

To 2.2g of 6,11-dihydro-11-ethyl-5H-pyrido[2,3-b][1,5]benzodiazepin-5-one, prepared as in Example 21, was added 1.9g of Lawesson's Reagent and 50 ml of toluene. The mixture was heated to reflux for 2 1/2 hours and then cooled to room temperature. The reaction mixture was concentrated in vacuo and the solid residue was dissolved in methylene chloride. The resulting solid was filtered and recrystallized from ethyl acetate to yield 0.66g (28% of theory) of 6,11-dihydro-11-ethyl-5H-pyrido [2,3-b][1,5]benzodiazepin-5-thione, m.p. 194-196°C.

Example 25

6,11-Dihydro-11-benzyl-6-methyl-5H-pyrido[2,3-b][1,5]benzodiazepin-5-one

To a mixture of 4.0g of 6,11-dihydro-6-methyl-5H-pyrido[2,3-b] [1,5]benzodiazepin-5-one and 100 ml of dry DMF was added 1.2g of 50% NaH in mineral oil. The resulting solution was heated to 60°C for one hour. The dark red solution was cooled to 30°C and 2.5 ml of benzyl bromide was added and stirred overnight under nitrogen. Methanol was added slowly until bubbling ceased, and the reaction mixture was then poured into 400 ml of water. The product

was extracted with ether, dried over $MgSO_4$, then concentrated and purified by column chromatography over silica gel. Elution of the material with 2% ethyl acetate/methylene chloride gave a white solid, which was recrystallized from methylene chloride/ether/petroleum ether to give 2.2g (39% of theory) of 6,11-dihydro-11-benzyl-6-methyl-5H-pyrido [2,3-b][1,5]benzodiazepin-5-one, m.p. 168-170°C.

Example 26

6,11-Dihydro-11-(t-butoxycarbonyl)methyl-6-methyl-5H-pyrido[2,3-b][1,5] benzodiazepin-5-one

To 0.8g (3.5 mmoles) of 6,11-dihydro-6-methyl-5H-pyrido[2,3-b] [1,5]benzodiazepin-5-one in 75 ml of dimethylformamide was added 0.2g (4.0 mmol) of 50% sodium hydride in oil, and the reaction mixture was stirred at 60°C for 2h. It was then cooled to room temperature and 0.78g (4.0 mmoles) of t-butyl bromoacetate was added dropwise, and the reaction mixture was allowed to stir overnight. Excess sodium hydride was carefully quenched with water and the solvent was evaporated. The residue was dissolved in methylene chloride, washed with water and dried over anhydrous sodium sulfate. Evaporation of the solvent gave 2.0g of crude product. Purification by column chromatography (silica gel) using 10% ethyl acetate/methylene chloride afforded 0.52g of pure 6,11-Dihydro-11-(t-butoxycarbonyl)methyl-6-methyl-5H-pyrido [2,3-b][1,5]benzodiazepin-5-one, m.p. 64-65°C.

Example 27

6,11-Dihydro-11-(ethoxycarbonyl)methyl-6-methyl-5H-pyrido[2,3-b][1,5] benzodiazepin-5-one

To 1.0g (4.4mmoles) of 6,11-dihydro-6-methyl-5H-pyrido[2,3-b][1,5] benzodiazepin-5-one in 50 ml of dimethylformamide was added 0.25g (5 mmol) of 50% sodium hydride in oil. The reaction mixture was then stirred at 50°C for an additional 2h and then 0.6 ml (5mmol) of ethyl bromoacetate was added. The reaction mixture was then stirred at 50°C for an additional 3h. It was then cooled to room temperature and the solvent was evaporated. The residue was dissolved in methylene chloride, washed with water, dried over anhydrous sodium sulfate and evaporated to obtain 2.0g of the crude product. It was further purified by column chromatography (silica gel) using 10% ethyl acetate/methylene chloride as eluent to obtain 250mg of pure 6,11-dihydro-11-(ethoxy-carbonyl)methyl-6-methyl-5H-pyrido[2,3-b][1,5]benzodiazepin-5-one, m.p. 99-100°C.

Example 28

6,11-Dihydro-6,8,9-trimethyl-5H-pyrido[2,3-b][1,5]benzodiazepin-5-one

To a stirred suspension of 4.55 (0.202m) of 6,11-dihydro-8,9-dimethyl-5H-pyrido[2,3-b][1,5]benzodiazepin-5-one in 40ml of dimethylsulfoxide was added 3.0ml of a 30% NaOH solution (0.022mol). After two hours at room temperature 5.0 ml (0.08mol) of methyl iodide was added and the mixture stirred at room temperature overnight. It was then poured into water, the resulting precipitate collected, washed, dried and crystallized from ethanol to yield 3.3g (69% of theory) of a crystallized solid melting at 206-209°C.

Example 29

6,11-Dihydro-11-ethyl-6,8,9-trimethyl-5H-pyrido[2,3-b][1,5]benzodiazepin-5-one

Using a procedure analogous to that described in Example 2, 6,11-dihydro-11-ethyl 6,8,9-trimethyl-5H-pyrido [2,3-b][1,5]benzodiazepin-5-one, m.p. 140-144°C (crystallized from petroleum ether) was prepared from 6,11-dihydro-6,8,9-trimethyl-5H-pyrido[2,3-b][1,5]benzodiazepin-5-one, prepared as in Example 28, and methyl iodide. The yield was 41% of the theory.

Example 30

6,11-Dihydro-8-methoxycarbonyl-11-ethyl-6-methyl-5H-pyrido[2,3-b][1,5] benzodiazepin-5-one

a) 6,11-Dihydro-8-methoxycarbonyl-5H-pyrido[2,3-b][1,5] benzodiazepin-5-one

A stirred mixture of 1.66g (0.010mol) of 4-methoxycarbonyl-o-phenylenediamine and 1.58g (0.010mol) of 2-chloronicotinic acid in 5ml of 2-butoxyethanol was heated to 140°C for 2h during which time a dark solution, followed by

formation of a green solid, was observed. The reaction mixture was poured into 50ml of water and the solid collected and stirred in 10% sodium carbonate solution for 1h. The resulting solid was collected, washed thoroughly with water, dried and crystallized from DMF to yield 1.1g (40% of theory) of a beige crystalline product, m.p. 344-349°C.

b) 6,11-Dihydro-8-methoxycarbonyl-6-methyl-5H-pyrido[2,3-b][1,5] benzodiazepin-5-one

Using a procedure analogous to that described in Example 28 6,11-dihydro-8-methoxycarbonyl-6-methyl-5H-pyrido[2,3-b][1,5]benzodiazepin-5-one, m.p. 273-276°C (after trituration with ethanol) was prepared from 6,11-dihydro-8-methoxycarbonyl-5H-pyrido[2,3-b][1,5]benzodiazepin-5-one and methyl iodide. The yield was 74% of theory.

c) 6,11-dihydro-8-methoxycarbonyl-11-ethyl-6-methyl-5H-pyrido[2,3-b][1,5]benzodiazepin-5-one.

Using a procedure analogous to that described in Example 28 6,11-dihydro-8-methoxycarbonyl-11-ethyl-6-methyl-5H-pyrido[2,3-b] [1,5]benzodiazepin-5-one, m.p. 137-140°C (crystallized from petroleum ether, (60-90°) was prepared from 6,11-dihydro-8-methoxycarbonyl- 6-methyl-5H-pyrido[2,3-b][1,5]benzodiazepin-5-one and ethyl iodide. The yield was 32% of theory.

Example 31

6,11-Dihydro-6-ethyl-7-methyl-5H-pyrido[2,3-b][1,5]benzodiazepin-5-one

To a stirred suspension of 1.1g (5mmol) 6,11-dihydro-7-methyl-5H-pyrido [2,3-b][1,5]benzodiazepin-5-one in 20ml of dry DMF 0.13g (5.5mmol) of sodium hydride was added portionwise at room temperature. Once the evolution of hydrogen had ceased, the mixture was heated to 60°C for 2h, cooled to 10°C, and then 0.86g (5.5mmol) of ethyl iodide was added. The reaction mixture was stirred at room temperature overnight and then concentrated. The residue was poured into water, the solid collected, washed, dried and column chromatographed on 60g of dry silica gel. The product (second fraction) was eluted with methylene chloride and methylene chloride/methanol 99/1 to give 0.6g (80% of theory) of 6,11-Dihydro-6-ethyl-7-methyl-5H-pyrido[2,3-b][1,5]benzodiazepin-5-one m.p. 186-188°C.

Example 32

6,11-Dihydro-7-methyl-5H-pyrido[2,3-b][1,5]benzodiazepin-5-one

Using a procedure analogous to that described in Example 30, 6,11-dihydro-7-methyl-5H-pyrido[2,3-b][1,5]benzodiazepin-5-one, m.p. 233-235°C (crystallized from dioxane/water) was prepared from 3-methyl-o-phenylenediamine and 2-chloronicotinic acid. The yield was 35% of theory.

Example 33

6,11-Dihydro-11-(2-fluoroethyl)-6-methyl-5H-pyrido[2,3-6][1,5]benzodiazepin-5-one

Using a procedure analogous to that described in Example 2, 6,11- dihydro-11-(2-fluoroethyl)-6-methyl-5H-pyrido [2,3-b][1,5]benzodiazepin- 5-one, m.p. 118-120°C, was obtained (87% of theory) from 6,11-dihydro- 6-methyl-5H-pyrido[2,3-b][1,5]benzodiazepin-5-one and 1-bromo-2-fluoroethane.

Example 34

6,11-Dihydro-11-ethyl-6-fluoromethyl-5H-pyrido-[2,3-b][1,5]benzodiazepin- 5-one

a) 6,11-Dihydro-6-fluoromethyl-5H-pyrido[2,3-b][1,5]benzodiazepin-5-one Using a procedure analogous to that described in Example 2, 6,11-dihydro- 6-fluoromethyl-5H-pyrido[2,3-b][1,5]benzodiazepin-5-one, m.p. 108-110°C, was obtained (3% of theory) from 6,11-dihydro-5H-pyrido[2,3-b][1,5] benzodiazepin-5-one and bromofluoromethane.

b) 6-11-Dihydro-11-ethyl-6-fluoromethyl-5H-pyrido[2,3-b][1,5]benzo- diazepin-5-one
Using a procedure analogous to that described in Example 2, 6,11-dihydro- 11-ethyl-6-fluoromethyl-5H-pyrido [2,3-b][1,5]benzodiazepin-5-one, m.p. 113-116°C, was obtained (69% of theory) from 6,11-dihydro-6- fluoromethyl-5H-pyrido[2,3-b][1,5]benzodiazepin-5-one and ethyl iodide.

Example 35

6,11-Dihydro-11-ethyl-6-methyl-9-nitro-5H-pyrido[2,3-b][1,5]benzodiazepin-5-one

a) A mixture of 2-chloronicotinic acid (15.7g, 0.1 mol) and 4-nitrophenylene diamine (15.3g, 0.1 mol) was heated in sulfonane (100ml) at 170°C for 5 hrs. The reaction mixture was then cooled and left standing overnight. The solid material was filtered and taken up in boiling ethanol. The solid was filtered again an dried giving 21g (82% theory) of 6,11-dihydro-9-nitro-5H-pyrido[2,3-b][1,5]benzodiazepin-5-one suitable for use in the next reaction.

b) Sodium hydride (50% dispersion in oil, 4.0g, 83 mmoles) was placed in a 3-neck round bottom flask. After washing the sodium hydride three times with hexane, DMSO (200 mL) was added. The mixture was heated to 50°C until a clear solution was obtained and 21g of the product obtained in step a) was added as a solution/ suspension in DMSO (100mL). The mixture was stirred for 1 hour at which time methyl iodide (12g, 80 mmoles) was added, and the mixture was stirred at room temperature overnight. Water (200mL) was added to the reaction mixture and the precipitate filtered. The solid material was treated twice with 300mL portions of boiling ethanol for one hour and the combined filtrates were left standing at room temperature. Three crops of crystals were deposited over 4 days yielding 6.7g (25% of theory) of 6,11-dihydro-6-methyl-9-nitro-5H-pyrido[2,3-b][1,5]benzodiazepin-5-one m.p. 275-278°C.

c) To sodium hydride (50% in oil, 0.27g, 5.6 mmoles) was added THF 1mL. The mixture was swirled, the THF removed by pipette. DMSO (5mL) was added and the mixture stirred and heated at 50° C for 30 min. The product obtained in step b) (1.5g, 5.5 mmoles) was added as a solution/suspension in DMSO (5 mL) and stirred for 20 min. Ethyl iodide (0.98g, 6.3 mmoles) was added and the mixture was stirred an additional 30 min. The reaction was quenched with water (100 mL) and extracted with methylene chloride. The organic phase was washed with water three times, dried ($MgSO_4$), filtered and concentrated in vacuo. Column chromatography over silica gel (Eluent methylene chloride ethanol:99/1) gave 0.5g (30% of theory) of 6-11-dihydro-11-ethyl-6-methyl-9-nitro-5H-pyrido[2,3-b][1,5]benzodiazepin-5-one m.p. 179-180.5, after recrystallization from ethanol/hexane.

Example 36

6,11-Dihydro-9-amino-11-ethyl-6-methyl-5H-pyrido[2,3b][1,5]benzodiazepin-5-one.

To a solution of 0.3g (1mmol) of 6,11-dihydro-11-ethyl-6-methyl-9-nitro-5H-pyrido[2,3-b][1,5]benzodiazepin-5-one in acetic acid (5mL) was added a solution of $SnCl_2.2H_2O$ (1.7g) in concentrated hydrochloric acid (2.2 ml). The mixture was stirred at room temperature for 6 hours, and then poured into a saturated sodium bicarbonate solution and basified with aqueous sodium hydroxide. The aqueous phase was then extracted with ethyl acetate, dried ($MgSO_4$), filtered and concentrated in vacuo. The solid material obtained was recrystallized from ethyl acetate to give 0.12g (44% of theory) of 6,11-dihydro-9-amino-11-ethyl-6-methyl-5Hpyrido[2,3-b][1,5]benzodiazepin-5-one, m.p. 193-195°C.

| EXAMPLE A | | | |
|---|---|---|---|
| Capsules or Tablets | | | |
| A-1 | | A-2 | |
| Ingredients | Quantity | Ingredients | Quantity |
| Compound of Example 2 | 50 mg | Compound of Example 2 | 50 mg |
| Starch | 160 mg | Dicalcium Phosphate | 160 mg |
| Microcrys, Cellulose | 90 mg | Microcrys. Cellulose | 90 mg |
| Sodium Starch Gluctate | 10 mg | Steeric acid | 5 mg |
| Magnesium Stearate | 2 mg | Sodium Starch Glycolate | 10 mg |
| Fumed colloidal silica | 1 mg | Fumed colloidal silica | 1 mg |

| EXAMPLE B | |
|---|---|
| Parenteral Solutions | |
| Ingredients | Quantity |
| Compounds of Example 2 | 500mg |
| Ethanol | 25ml |
| Water for injection | q.s. to 100ml |

Compound of Example 2 is added to the ethanol and mixed until the solution is clear. Water is added and the resulting solution is then filtered into the appropriate vials or ampoules and sterilized by autoclaving.

| EXAMPLE C | |
|---|---|
| Nasal Solutions | |
| Ingredients | Quantity |
| Compound of Example 2 | 500mg |
| Propylene glycol | 30ml |
| Benzalkoniujm chloride | 200mg |
| EDTA | 200mg |
| Water | q.s. to 100ml |

The excipient materials are mixed and thereafter the compound of Example 2 is added and mixing is continued until the solution is clear. The water is added and the resulting solution is then filtered into the appropriate vials or ampoules.

## Claims

**1.** A compound of the formula I

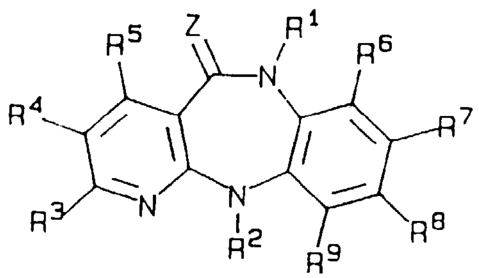

(I)

wherein,

Z is oxygen or sulfur;

$R^1$ is hydrogen, alkyl or fluoroalkyl of 1 to 5 carbon atoms, cyclopropyl, alkenyl or alkynyl of 2 to 5 carbon atoms, 2-halo-propen-1-yl, arylmethyl (wherein the aryl moiety is phenyl, thienyl or furanyl, which is either unsubstituted or substituted by methyl methoxy or halogen), alkanoyl of 2 to 3 carbon atoms, or alkoxyalkyl or alkylthioalkyl of 2 to 4 carbon atoms;

$R^2$ is alkyl or fluoroalkyl of 1 to 5 carbon atoms, cycloalkyl of 3 to 5 carbon atoms, alkenyl or alkynyl of 2 to 5 carbon atoms, alkoxyalkyl or alkylthioalkyl of 2 to 4 carbon atoms, alkanoyl of 2 to 4 carbon atoms, hydroxyalkyl of 2 to 5 carbon atoms, arylmethyl (wherein the aryl moiety is phenyl, thienyl or furanyl, which is either unsubstituted or substituted by alkyl or alkoxy of 1 to 3 carbon atoms, hydroxyl, or halogen), phenyl (which is either

unsubstituted or substituted by alkyl or alkoxy of 1 to 3 carbon atoms, halogen or hydroxyl) or alkoxycarbonylmethyl wherein the alkoxy moiety contains 1 to 5 carbon atoms;

$R^3$, $R^4$, and $R^5$ are each independently hydrogen or alkyl of 1 to 3 carbon atoms, with the proviso that at least one of these substituents is hydrogen; or,

one of $R^3$, $R^4$ and $R^5$ is butyl, alkanoyl of 1 to 3 carbon atoms, hydroxyalkyl of 1 to 4 carbon atoms, alkoxycarbonyl of 2 to 3 carbon atoms, alkoxycarbonylalkyl wherein both the alkoxy and alkyl moieties contain 1 to 2 carbon atoms, halogen, trihalomethyl, hydroxyl, alkoxy of 1 to 3 carbon atoms, alkythio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido or mono- or di-alkylaminoalkyl wherein the alkyl moieties each contain 1 to 2 carbon atoms, and the remaining two substituents are hydrogen or methyl; or,

when Z is oxygen, one of $R^3$, $R^4$ and $R^5$ is alkylsulfinyl or alkylsulfonyl of 1 to 3 carbon atoms, with the proviso that the remaining two substituents are hydrogrogen or methyl; and

$R^6$, $R^7$, $R^8$ and $R^9$ are each hydrogen; or,

one of $R^6$, $R^7$, $R^8$ and $R^9$ is alkyl of 1 to 4 carbon atoms, alkanoyl of 1 to 3 carbon atoms, alkoxycarbonyl of 2 to 3 carbon atoms, hydroxyalkyl of 1 to 4 carbon atoms alkoxycarbonylalkyl wherein both the alkoxy and alkyl moieties contain 1 to 2 carbon atoms, halogen, trihalomethyl, hydroxyl, alkoxy of 1 to 3 carbon atoms, alkylthio of 1 to 3 carbon atoms, alkanoyloxy of 2 to 3 carbon atoms, alkanoylamino of 1 to 3 carbon atoms, aminoalkyl of 1 to 3 carbon atoms, mono- or di-alkylamino wherein each alkyl moiety contains 1 to 2 carbon atoms, carboxyalkyl of 2 to 3 carbon atoms, cyano, nitro, carboxyl, carbamyl, amino, azido or mono- or di-alkylaminoalkyl wherein each alkyl moiety contains 1 to 2 carbon atoms, and the remaining three substituents are hydrogen or two of the remaining three substituents are hydrogen and one is methyl, ethyl or halogen;

or a pharmaceuticall acceptable acid addition salt thereof.

**2.** A compound of formula I, as set forth in claim 1, wherein,

Z is oxygen or sulfur;

$R^1$ is hydrogen, alkyl of 1 to 4 carbon atoms, alkenyl or alkynyl of 2 to 4 carbon atoms, 2-halo-propen-1-yl, or alkoxyalkyl or alkylthioalkyl of 2 to 3 carbon atoms;

$R^2$ is alkyl of 1 to 4 carbon atoms, cycloalkyl of 3 to 4 carbon atoms, alkenyl or alkynyl of 2 to 4 carbon atoms, alkoxyalkyl or alkylthioalkyl of 2 to 4 carbon atoms, alkanoyl of 2 to 3 carbon atoms, hydroxyalkyl of 2 to 4 carbon atoms, arylmethyl (wherein the aryl moiety is phenyl or thienyl, which is either unsubstituted or substituted by methyl, methoxy, hydroxyl or halogen), phenyl (which is either unsubstituted or substituted with methyl, methoxy, hydroxyl or halogen) or alkoxycarbonylmethyl wherein the alkoxy moiety contains 1 to 5 carbon atoms;

$R^3$, $R^4$, and $R^5$ are each independently hydrogen or methyl, with the proviso that at least one of these substituents is hydrogen, or $R^5$ is ethyl, propyl, or butyl with the other two substituents being hydrogen;

$R^6$ is hydrogen, methyl or ethyl with the proviso that $R^7$ is hydrogen or methyl;

$R^7$ is alkyl of 1 to 3 carbon atoms, alkanoyl of 1 to 3 carbon atoms, alkoxycarbonyl of 1 to 3 carbon stoms, hydroxyalkyl of 1 to 3 carbon atoms, alkoxycarbonylalkyl wherein the alkoxy and alkyl moieties each contain 1 to 2 carbon atoms, halogen, trifluoromethyl, hydroxyl, alkoxy or alkylthio of 1 to 2 carbon atoms, acetyloxy, alkanoylamino or aminoalkyl of 1 to 2 carbon atoms, cyano, nitro, amino, or mono- or di-methyl or -ethylamino, with the proviso that $R^8$ is hydrogen;

$R^8$ is alkyl of 1 to 3 carbon atoms, alkanoyl of 1 to 3 carbon atoms, alkoxycarbonyl of 1 to 3 carbon atoms, hydroxyalkyl of 1 to 3 carbon atoms, alkoxycarbonylalkyl wherein the alkoxy and alkyl moieties each contain 1 to 3 carbon atoms, halogen, trifluoromethyl, hydroxyl, alkoxy or alkylthio of 1 to 2 carbon atoms, acetyloxy,

alkanoylamino or aminoalkyl of 1 to 2 carbon atoms, cyano, nitro, amino, or mono- or di-methyl or -ethylamino with the proviso that $R^7$ is hydrogen; or,

when Z is oxygen and $R^8$ is hydrogen or methyl, $R^7$ may additionally be alkylsulfinyl or alkylsulfonyl of 1 to 2 carbon atoms, and when Z is oxygen and $R^7$ is hydrogen or methyl, $R^8$ may additionally be alkylsulfinyl or alkylsulfonyl of 1 to 2 carbon atoms; or,

$R^7$ and $R^8$ are both hydrogen, methyl or halogen; and,

$R^9$ is hydrogen or methyl with the proviso that $R^8$ is hydrogen or methyl:

or a pharmaceutically acceptable acid addition salt thereof.

**3.** A compound of formula 1, as set forth in claim 1, wherein,

Z is oxygen or sulfur;

$R^1$ is hydrogen, alkyl of 1 to 3 carbon atoms, 2-halo-2-propen-1-yl, or alkoxyalkyl or alkylthioalkyl of two to three carbon atoms;

$R^2$ is alkyl of 1 to 4 carbon atoms, cycloalkyl of 3 to 4 carbon atoms, alkenyl or alkynyl of 2 to 4 carbon atoms, alkyloxyalkyl or alkylthioalkyl of 2 to 3 carbon atoms, alkanoyl of 2 to 3 carbon atoms, hydroxyalkyl of 2 to 4 carbon atoms, arylmethyl (wherein the aryl moiety is phenyl or thienyl, which is either unsubstituted or substituted by methyl, methoxy or halogen) or alkoxycarbonylmethyl wherein the alkoxy moiety contains 1 to 5 carbon atoms; and,

$R^3$ through $R^9$ are as set forth belox in Table A;

or a pharmaceutically acceptable acid addition salt thereof,

TABLE A

| | $R^3$ | $R^4$ | $R^5$ | $R^6$ | $R^7$ | $R^8$ | $R^9$ |
|---|---|---|---|---|---|---|---|
| a | H | H | H | H | H | $CF_3$ | H |
| b | H | H | H | H | Cl | H | H |
| c | H | H | H | H | $CH_3$ | $CH_3$ | H |
| d | $CH_3$ | H | H | H | $CH_3$ | $CH_3$ | H |
| e | $CH_3$ | $CH_3$ | H | H | $CH_3$ | $CH_3$ | H |
| f | H | H | H | H | $CH_3$ | $CH_3$ | H |
| g | H | H | H | H | H | Cl | H |
| h | H | H | H | H | H | H | H |
| i | H | H | H | $CH_3$ | H | H | H |
| j | H | H | H | H | $CH_3O_2C$- | H | H |
| k | H | H | H | H | $C_2H_5O_2C$- | H | H |
| l | H | H | H | H | NC- | H | H |
| m | H | H | H | H | $CH_3CO$- | H | H |
| n | H | H | H | H | H | $CH_3O_2C$- | H |
| o | H | H | H | H | H | $C_2H_5O_2C$- | H |
| p | H | H | H | H | H | NC- | H |
| q | H | H | H | H | H | $CH_3CO$- | H |

**4.** A compound of formula I, as set forth in claim 1, wherein,

Z is oxygen or sulfur;

$R^1$ is hydrogen, alkyl of 1 to 2 carbon atoms or allyl;

$R^2$ is alkyl of 2 to 3 carbon atoms, cyclopropyl or allyl; and

$R^3$ through $R^9$ are each hydrogen or $R^7$ and $R^8$ are both methyl or chloro and $R^3$, $R^4$, $R^5$, $R^6$ and $R^9$ are each hydrogen;

or a pharmaceutically acceptable salt thereof.

**5.** A compound of formula I, in accordance with claim 1, selected from the group consisting of the following:

a) 6,11-dihydro-11-ethyl-6-methyl-5H-pyrido[2,3-b][1,5]benzodiazepin-5-one,

b) 6,11-dihydro-6-methyl-11-propyl-5H-pyrido[2,3-b][1,5]benzodiazepin-5-one;

c) 6,11-dihydro-6-methyl-11-methylethyl-5H-pyrido[2,3-b][1,5]benzodiazepin-5-one;

d) 6,11-dihydro-11-ethyl-6-methyl-5H-pyrido[2,3-b][1,5]benzodiazepin-5-one;

e) 6,11-dihydro-11-ethyl-5H-pyrido[2,3-b][1,5]benzodiazepin-5-one;

f) 6,11-dihydro-11-ethyl-5H-pyrido[2,3-b][1,5]benzodiazepin-5-thione;

g) 6,11-dihydro-6,8,9,11-tetramethyl-5H-pyrido[2,3-b][1,5]benzodiazepin-5-one;

h) 6,11-dihydro-6-ethyl-11-methyl-5H-pyrido[2,3-b][1,5]benzodiazepin-5-one;

i) 6,11-dihydro-6-n-propyl-11-methyl-5H-pyrido[2,3-b][1,5]benzodiazepin-5-one;

j) 6,11-dihydro-6-i-propyl-11-methyl-5H-pyrido[2,3-b][1,5]benzodiazepin-5-one;

k) 6,11-dihydro-6,11-diethyl-5H-pyrido[2,3-b][1,5]benzodiazepin-5-one;

l) 6,11-dihydro-11-ethyl-5H-pyrido[2,3-b][1,5]benzodiazepin-5-thione;

m) 6,11-dihydro-11-ethyl-6,8,9-trimethyl-5H-pyrido[2,3-b][1,5]benzodiazepin-5-one;

n) 6,11-dihydro-8-methoxycarbonyl-11-ethyl-6-methyl-5H-pyrido[2,3-b][1,5]benzodiazepin-5-one; and,

o) 6,11-dihydro-11-fluoroethyl-6-methyl-5H-pyrido[2,3-b][1,5]benzodiazepin-5-one.

**6.** 6,11-dihydro-11-ethyl-6-methyl-5H-pyrido[2,3-b][1,5] benzodiazepin-5-one.

**7.** The use of a compound according to claims 1, 2, 3, 4, 5 or 6 in the manufacture of a pharmaceutical preparation for the prophylaxis or treatment of a human exposed to or infected by HIV-1.

**8.** A pharmaceutical composition for the prevention or treatment of HIV-1 infection comprising a compound according to anyone of claims 1, 2, 3, 4, 5 or 6 and a pharmaceutically acceptable carrier.

**9.** The use of a compound selected from the group consisting of:

a) 2,4,6,8 tetramethyl-6-11-dihydro-5H-pyrido[2,3-b] [1,5]-benzodiazepin-5-one or -thione;

b) 6-methyl-6,11-dihydro-5H-pyrido[2,3-b][1,5]benzodiazepin-5-one or -thione;

c) 6-11-dihydro-5H-pyrido[2,3-b][1,5]benzodiazepin-5-one or -thione;

d) 6-ethyl-6,11-dihydro-5H-pyrido[2,3-b][1,5]benzodiazepin-5-one or -thione;

e) 6,8,9-trimethyl-6,11-dihydro-5H-pyrido[2,3-b][1,5]benzodiazepin-5-one or -thione;

f) 6-ethyl-8,9-dimethyl-6,11-dihydro-5H-pyrido[2,3-b] [1,5]-benzodiazepin-5-one or -thione;

g) 6-isobutyl-6,11-dihydro-5H-pyrido[2,3-b][1,5]benzodiazepin-5-one or -thione;

and
h) 6-ethyl-9-methyl-6,11-dihydro-5H-pyrido[2,3-b][1,5]benzodiazepin-5-one or -thione
in the manufacture of a pharmaceutical preparation for the prophylaxis or treatment of a human exposed to or infected by HIV-1.

**10.** A compound selected from the group consisting of the following:

a) 2,4,6,8 tetramethyl-6,11-dihydro-5H-pyrido[2,3-b] [1,5]-benzodiazepin-5-thione;

b) 6-methyl-6,11-dihydro-5H-pyrido[2,3-b][1,5]benzodiazepin-5-thione;

c) 6,11-dihydro-5H-pyrido[2,3-b][1,5]benzodiazepin-5-thione;

d) 6-ethyl-6,11-dihydro-5H-pyrido[2,3-b][1,5]benzodiazepin-5-thione;

e) 6,8,9-trimethyl-6,11-dihydro-5H-pyrido[2,3-b][1,5]benzodiazepin-5-thione;

f) 6-ethyl-8,9-dimethyl-6,11-dihydro-5H-pyrido[2,3-b] [1,5]-benzodiazepin-5-thione;

g) 6-isobutyl-6,11-dihydro-5H-pyrido[2,3-b] [1,5]benzodiazepin-5-thione;

and
h) 6-ethyl-9-methyl-6,11-dihydro-5H-pyrido[2,3-b][1,5]benzodiazepin-5-thione;
and pharmaceutically acceptable salts thereof.

**11.** A method for the preparation of a compound as defined in Claim 1 or Claim 10, comprising one of the following methods,

A) for preparing a compound of formula 1 wherein Z is oxygen and both $R^1$ and $R^2$ are other than hydrogen, converting a 6,11-dihydro-5H-pyrido[2,3-b][1,5]benzodiazepin-5-one of the formula II

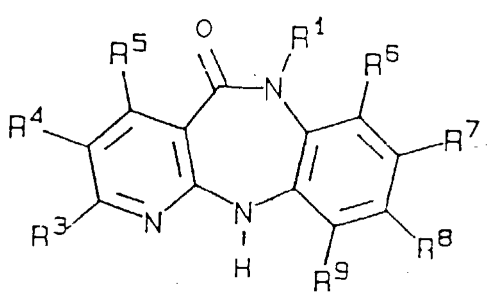

II

wherein $R^1$ and $R^3$ to $R^9$ have the same meanings as set forth with respect to Formula 1 but $R^1$ is other than H, into the corresponding 11-alkali metal compound, and subsequently reacting the alkali metal compound with a compound of the formula III

$$R^2X \qquad III$$

wherein $R^2$ has the same meanings set forth with respect to Formula 1 but is other than hydrogen, and X is

a suitable leaving group,

B) for preparing a compound of Formula 1 wherein Z is oxygen and $R^1$ is hydrogen, hydrolysing a compound of Formula IV

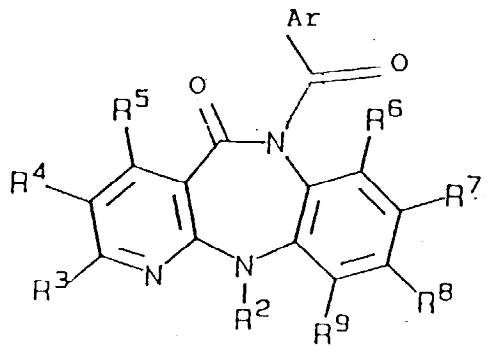

IV

wherein the groups $R^2$ to $R^9$ are as set forth with respect to Formula 1 and Ar is an aromatic or heterocyclic group consisting of one or two nuclei which is or are optionally substituted by halogen, methyl of methoxy; or

C) for preparing a compound of Claim 10 or a 5-thione of Formula I, by reacting a 5-one compound corresponding to the desired compound of Claim 11, or a 5-one compound of Formula I, with a sulfurating agent,

and thereafter isolating the resulting 5-one or 5-thione obtained from method A, B or C as such or as an acid addition salt.

**12.** A method for the preparation of a pharmaceutical composition which comprises mixing a compound according to Claim 1 or Claim 10 with a pharmaceutically acceptable carrier or excipient.

## Patentansprüche

**1.** Verbindung der Formel I

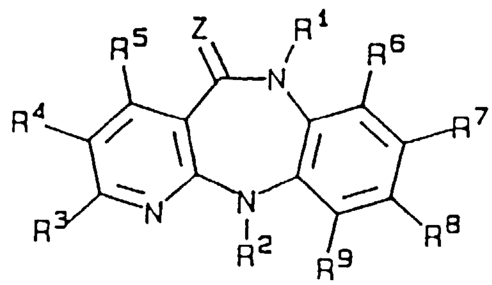

(I)

mit den nachfolgenden Bedeutungen:

Z bedeutet Sauerstoff oder Schwefel;

$R^1$ bedeutet Wasserstoff, Alkyl oder Fluoralkyl mit 1 bis 5 Kohlenstoffatomen, Cyclopropyl, Alkenyl oder Alkynyl mit 2 bis 5 Kohlenstoffatomen, 2-Halopropen-1-yl, Arylmethyl (worin der Aryl-Molekülteil ein entweder unsubstituiertes oder mit Methyl, Methoxy oder Halogen substituiertes Phenyl, Thienyl oder Furanyl ist), Alkanoyl mit 2 bis 3 Kohlenstoffatomen, oder Alkoxyalkyl oder Alkylthioalkyl mit 2 bis 4 Kohlenstoffatomen;

$R^2$ bedeutet Alkyl oder Fluoralkyl mit 1 bis 5 Kohlenstoffatomen, Cycloalkyl mit 3 bis 5 Kohlenstoffatomen,

Alkenyl oder Alkynyl mit 2 bis 5 Kohlenstoffatomen, Alkoxyalkyl oder Alkylthioalkyl mit 2 bis 4 Kohlenstoffatomen, Alkanoyl mit 2 bis 4 Kohlenstoffatomen, Hydroxyalkyl mit 2 bis 5 Kohlenstoffatomen, Arylmethyl (worin der Aryl-Molekülteil ein entweder unsubstituiertes oder mit Alkyl oder Alkoxy mit 1 bis 3 Kohlenstoffatomen, Hydroxyl oder Halogen substituiertes Phenyl, Thienyl oder Furanyl ist), Phenyl (das entweder unsubstituiert oder mit Alkyl oder Alkoxy mit 1 bis 3 Kohlenstoffatomen, Halogen oder Hydroxyl substituiert ist), oder Alkoxycarbonylmethyl, worin der Alkoxy-Molekülteil 1 bis 5 Kohlenstoffatome enthält;

$R^3$, $R^4$ und $R^5$ bedeuten unabhängig voneinander Wasserstoff oder Alkyl mit 1 bis 3 Kohlenstoffatomen mit der Massgabe, dass mindestens einer dieser Substituenten Wasserstoff ist; oder

einer von $R^3$, $R^4$ und $R^5$ bedeutet Butyl, Alkanoyl mit 1 bis 3 Kohlenstoffatomen, Hydroxyalkyl mit 1 bis 4 Kohlenstoffatomen, Alkoxycarbonyl mit 2 bis 3 Kohlenstoffatomen, Alkoxycarbonylalkyl, worin der Alkoxy-Molekülteil und der Alkyl-Molekülteil beide jeweils 1 bis 2 Kohlenstoffatome enthalten, Halogen, Trihalomethyl, Hydroxyl, Alkoxy mit 1 bis 3 Kohlenstoffatomen, Alkylthio mit 1 bis 3 Kohlenstoffatomen, Alkanoyloxy mit 2 bis 3 Kohlenstoffatomen, Alkanoylamino mit 1 bis 3 Kohlenstoffatomen, Aminoalkyl mit 1 bis 3 Kohlenstoffatomen, Mono- oder Dialkylamino, worin jeder Alkyl-Molekülteil 1 bis 2 Kohlenstoffatome enthält, Carboxyalkyl mit 2 bis 3 Kohlenstoffatomen, Cyano, Nitro, Carboxyl, Carbamyl, Amino, Azido, oder Mono- oder Dialkylaminoalkyl, worin jeder Alkyl-Molekülteil 1 bis 2 Kohlenstoffatome enthält, und die verbleibenden Substituenten Wasserstoff oder Methyl sind; oder

wenn Z Sauerstoff bedeutet, bedeutet einer von $R^3$, $R^4$ und $R^5$ Alkylsulfinyl oder Alkylsulfonyl mit 1 bis 3 Kohlenstoffatomen mit der Massgabe, dass die beiden verbleibenden Substituenten Wasserstoff oder Methyl sind; und

jeder von $R^6$, $R^7$, $R^8$ und $R^9$ bedeutet Wasserstoff; oder

einer von $R^6$, $R^7$, $R^8$ und $R^9$ bedeutet Alkyl mit 1 bis 4 Kohlenstoffatomen, Alkanoyl mit 1 bis 3 Kohlenstoffatomen, Alkoxycarbonyl mit 2 bis 3 Kohlenstoffatomen, Hydroxyalkyl mit 1 bis 4 Kohlenstoffatomen, Alkoxycarbonylalkyl, worin der Alkoxy-Molekülteil und der Alkyl-Molekülteil beide jeweils 1 bis 2 Kohlenstoffatome enthalten, Halogen, Trihalomethyl, Hydroxyl, Alkoxy mit 1 bis 3 Kohlenstoffatomen, Alkylthio mit 1 bis 3 Kohlenstoffatomen, Alkanoyloxy mit 2 bis 3 Kohlenstoffatomen, Alkanoylamino mit 1 bis 3 Kohlenstoffatomen, Aminoalkyl mit 1 bis 3 Kohlenstoffatomen, Mono- oder Dialkylamino, worin jeder Alkyl-Molekülteil 1 bis 2 Kohlenstoffatome enthält, Carboxyalkyl mit 2 bis 3 Kohlenstoffatomen, Cyano, Nitro, Carboxyl, Carbamyl, Amino, Azido, oder Mono- oder Dialkylaminoalkyl, worin jeder Alkyl-Molekülteil 1 bis 2 Kohlenstoffatome enthält, und die verbleibenden drei Substituenten Wasserstoff sind oder zwei der drei verbleibenden Substituenten Wasserstoff sind und einer Methyl, Ethyl oder Halogen ist;

oder ein pharmazeutisch annehmbares Säureadditionssalz davon.

**2.** Verbindung der Formel I nach Anspruch 1 mit den nachfolgenden Bedeutungen:

Z bedeutet Sauerstoff oder Schwefel;

$R^1$ bedeutet Wasserstoff, Alkyl mit 1 bis 4 Kohlenstoffatomen, Alkenyl oder Alkynyl mit 2 bis 4 Kohlenstoffatomen, 2-Halopropen-1-yl, oder Alkoxyalkyl oder Alkylthioalkyl mit 2 bis 3 Kohlenstoffatomen;

$R^2$ bedeutet Alkyl mit 1 bis 4 Kohlenstoffatomen, Cycloalkyl mit 3 bis 4 Kohlenstoffatomen, Alkenyl oder Alkynyl mit 2 bis 4 Kohlenstoffatomen, Alkoxyalkyl oder Alkylthioalkyl mit 2 bis 4 Kohlenstoffatomen, Alkanoyl mit 2 bis 3 Kohlenstoffatomen, Hydroxyalkyl mit 2 bis 4 Kohlenstoffatomen, Arylmethyl (worin der Aryl-Molekülteil ein entweder unsubstituiertes oder mit Methyl, Methoxy, Hydroxyl oder Halogen substituiertes Phenyl oder Thienyl ist), Phenyl (das entweder unsubstituiert oder mit Methyl, Methoxy, Hydroxyl oder Halogen substituiert ist), oder Alkoxycarbonylmethyl, worin der Alkoxy-Molekülteil 1 bis 5 Kohlenstoffatome enthält;

$R^3$, $R^4$ und $R^5$ bedeuten unabhängig voneinander Wasserstoff oder Methyl mit der Massgabe, dass mindestens einer dieser Substituenten Wasserstoff ist, oder $R^5$ bedeutet Ethyl, Propyl oder Butyl und die beiden anderen Substituenten sind Wasserstoff;

$R^6$ bedeutet Wasserstoff, Methyl oder Ethyl mit der Massgabe, dass $R^7$ Wasserstoff oder Methyl bedeutet;

$R^7$ bedeutet Alkyl mit 1 bis 3 Kohlenstoffatomen, Alkanoyl mit 1 bis 3 Kohlenstoffatomen, Alkoxycarbonyl mit 1 bis 3 Kohlenstoffatomen, Hydroxyalkyl mit 1 bis 3 Kohlenstoffatomen, Alkoxycarbonylalkyl, worin der Alkoxy-Molekülteil und der Alkyl-Molekülteil beide jeweils 1 bis 2 Kohlenstoffatome enthalten, Halogen, Trifluormethyl, Hydroxyl, Alkoxy oder Alkylthio mit 1 bis 2 Kohlenstoffatomen, Acetyloxy, Alkanoylamino oder Aminoalkyl mit 1 bis 2 Kohlenstoffatomen, Cyano, Nitro, Amino, oder Mono- oder Dimethyl- oder -ethylamino, mit der Massgabe, dass $R^8$ Wasserstoff bedeutet;

$R^8$ bedeutet Alkyl mit 1 bis 3 Kohlenstoffatomen, Alkanoyl mit 1 bis 3 Kohlenstoffatomen, Alkoxycarbonyl mit 1 bis 3 Kohlenstoffatomen, Hydroxyalkyl mit 1 bis 3 Kohlenstoffatomen, Alkoxycarbonylalkyl, worin der Alkoxy-Molekülteil und der Alkyl-Molekülteil beide jeweils 1 bis 3 Kohlenstoffatome enthalten, Halogen, Trifluormethyl, Hydroxyl, Alkoxy oder Alkylthio mit 1 bis 2 Kohlenstoffatomen, Acetyloxy, Alkanoylamino oder Aminoalkyl mit 1 bis 2 Kohlenstoffatomen, Cyano, Nitro, Amino, oder Mono- oder Dimethyl- oder -ethylamino, mit der Massgabe, dass $R^7$ Wasserstoff bedeutet; oder

wenn Z Sauerstoff und $R^8$ Wasserstoff oder Methyl bedeutet, kann $R^7$ zusätzlich Alkylsulfinyl oder Alkylsulfonyl mit 1 bis 2 Kohlenstoffatomen bedeuten, und wenn Z Sauerstoff und $R^7$ Wasserstoff oder Methyl bedeutet, kann $R^8$ zusätzlich Alkylsulfinyl oder Alkylsulfonyl mit 1 bis 2 Kohlenstoffatomen bedeuten; oder

$R^7$ und $R^8$ bedeuten beide Wasserstoff, Methyl oder Halogen; und

$R^9$ bedeutet Wasserstoff oder Methyl mit der Massgabe, dass $R^8$ Wasserstoff oder Methyl bedeutet;

oder ein pharmazeutisch annehmbares Säureadditionssalz davon.

**3.** Verbindung der Formel I nach Anspruch 1 mit den nachfolgenden Bedeutungen:

Z bedeutet Sauerstoff oder Schwefel;

$R^1$ bedeutet Wasserstoff, Alkyl mit 1 bis 3 Kohlenstoffatomen, 2-Halo-2-propen-1-yl, oder Alkoxyalkyl oder Alkylthioalkyl mit 2 bis 3 Kohlenstoffatomen;

$R^2$ bedeutet Alkyl mit 1 bis 4 Kohlenstoffatomen, Cycloalkyl mit 3 bis 4 Kohlenstoffatomen, Alkenyl oder Alkynyl mit 2 bis 4 Kohlenstoffatomen, Alkoxyalkyl oder Alkylthioalkyl mit 2 bis 3 Kohlenstoffatomen, Alkanoyl mit 2 bis 3 Kohlenstoffatomen, Hydroxyalkyl mit 2 bis 4 Kohlenstoffatomen, Arylmethyl (worin der Aryl-Molekülteil ein entweder unsubstituiertes oder mit Methyl, Methoxy oder Halogen substituiertes Phenyl oder Thienyl ist), oder Alkoxycarbonylmethyl, worin der Alkoxy-Molekülteil 1 bis 5 Kohlenstoffatome enthält; und

$R^3$ bis $R^9$ haben die in Tabelle A angegebene Bedeutung:

Tabelle A

| | $R^3$ | $R^4$ | $R^5$ | $R^6$ | $R^7$ | $R^8$ | $R^9$ |
|---|---|---|---|---|---|---|---|
| a | H | H | H | H | H | $CF_3$ | H |
| b | H | H | H | H | Cl | H | H |
| c | H | H | H | H | $CH_3$ | $CH_3$ | H |
| d | $CH_3$ | H | H | H | $CH_3$ | $CH_3$ | H |
| e | $CH_3$ | $CH_3$ | H | H | $CH_3$ | $CH_3$ | H |
| f | H | H | H | H | $CH_3$ | $CH_3$ | H |
| g | H | H | H | H | H | Cl | H |
| h | H | H | H | H | H | H | H |
| i | H | H | H | $CH_3$ | H | H | H |
| j | H | H | H | H | $CH_3O_2C$- | H | H |
| k | H | H | H | H | $C_2H_5O_2C$- | H | H |
| l | H | H | H | H | NC- | H | H |
| m | H | H | H | H | $CH_3CO$- | H | H |
| n | H | H | H | H | H | $CH_3O_2C$- | H |

Tabelle A (fortgesetzt)

| | $R^3$ | $R^4$ | $R^5$ | $R^6$ | $R^7$ | $R^8$ | $R^9$ |
|---|---|---|---|---|---|---|---|
| o | H | H | H | H | H | $C_2H_5O_2C$- | H |
| p | H | H | H | H | H | NC- | H |
| q | H | H | H | H | H | $CH_3CO$- | H |

oder ein pharmazeutisch annehmbares Säureadditionssalz davon.

**4.** Verbindung der Formel I nach Anspruch 1 mit den nachfolgenden Bedeutungen:

Z bedeutet Sauerstoff oder Schwefel;

$R^1$ bedeutet Wasserstoff, Alkyl mit 1 bis 2 Kohlenstoffatomen oder Allyl;

$R^2$ bedeutet Alkyl mit 2 bis 3 Kohlenstoffatomen, Cyclopropyl oder Allyl; und

jeder von $R^3$ bis $R^9$ bedeutet Wasserstoff, oder $R^7$ und $R^8$ bedeuten beide Methyl oder Chlor und jeder von $R^3$, $R^4$, $R^5$, $R^6$ und $R^9$ bedeutet Wasserstoff;

oder ein pharmazeutisch annehmbares Salz davon.

**5.** Verbindung der Formel I nach Anspruch 1, ausgewählt aus der von den nachfolgenden Stoffen gebildeten Gruppe:

a) 6,11-Dihydro-11-ethyl-6-methyl-5H-pyrido[2,3-b][1,5]benzodiazepin-5-on;
b) 6,11-Dihydro-6-methyl-11-propyl-5H-pyrido[2,3-b][1,5]benzodiazepin-5-on;
c) 6,11-Dihydro-6-methyl-11-methylethyl-5H-pyrido[2,3-b][1,5]benzodiazepin-5-on;
d) 6,11-Dihydro-11-ethyl-6-methyl-5H-pyrido[2,3-b][1,5]benzodiazepin-5-on;
e) 6,11-Dihydro-11-ethyl-5H-pyrido[2,3-b][1,5]benzodiazepin-5-on;
f) 6,11-Dihydro-11-ethyl-5H-pyrido[2,3-b][1,5]benzodiazepin-5-thion;
g) 6,11-Dihydro-6,8,9,11-tetramethyl-5H-pyrido[2,3-b][1,5]benzodiazepin-5-on;
h) 6,11-Dihydro-6-ethyl-11-methyl-5H-pyrido[2,3-b][1,5]benzodiazepin-5-on;
i) 6,11-Dihydro-6-n-propyl-11-methyl-5H-pyrido[2,3-b][1,5]benzodiazepin-5-on;
j) 6,11-Dihydro-6-i-propyl-11-methyl-5H-pyrido[2,3-b][1,5]benzodiazepin-5-on;
k) 6,11-Dihydro-6,11-diethyl-5H-pyrido[2,3-b][1,5]benzodiazepin-5-on;
l) 6,11-Dihydro-11-ethyl-5H-pyrido[2,3-b][1,5]benzodiazepin-5-thion;
m) 6,11-Dihydro-ll-ethyl-6,8,9-trimethyl-5H-pyrido[2,3-b][1,5]benzodiazepin-5-on;
n) 6,11-Dihydro-8-methoxycarbonyl-11-ethyl-6-methyl-5H-pyrido[2,3-b][1,5]benzodiazepin-5-on; und
o) 6,11-Dihydro-ll-fluorethyl-6-methyl-5H-pyrido[2,3-b][1,5]benzodiazepin-5-on.

**6.** 6,11-Dihydro-11-ethyl-6-methyl-5H-pyrido[2,3-b][1,5]benzodiazepin-5-on.

**7.** Verwendung einer Verbindung nach den Ansprüchen 1, 2, 3, 4, 5 oder 6 bei der Herstellung eines Arzneimittels zur Prophylaxe oder Behandlung eines dem HIV-1-Virus ausgesetzten oder davon infizierten Menschen.

**8.** Arzneimittel zur Prävention oder Behandlung einer Infektion mit dem HIV-1-Virus, enthaltend eine Verbindung nach einem der Ansprüche 1, 2, 3, 4, 5 oder 6 und einen pharmazeutisch annehmbaren Träger.

**9.** Verwendung einer Verbindung, ausgewählt aus der von den nachfolgenden Stoffen gebildeten Gruppe:

a) 2,4,6,8-Tetramethyl-6,11-dihydro-5H-pyrido[2,3-b][1,5]benzodiazepin-5-on oder -thion;
b) 6-Methyl-6,11-dihydro-5H-pyrido[2,3-b][1,5]benzodiazepin-5-on oder -thion;
c) 6,11-Dihydro-5H-pyrido[2,3-b][1,5]benzodiazepin-5-on oder -thion;
d) 6-Ethyl-6,11-dihydro-5H-pyrido[2,3-b][1,5]benzodiazepin-5-on oder -thion;
e) 6,8,9-Trimethyl-6,11-dihydro-5H-pyrido[2,3-b][1,5]benzodiazepin-5-on oder -thion;
f) 6-Ethyl-8,9-dimethyl-6,11-dihydro-5H-pyrido[2,3-b][1,5]benzodiazepin-5-on oder -thion;
g) 6-Isobutyl-6,11-dihydro-5H-pyrido[2,3-b][1,5]benzodiazepin-5-on oder -thion;

und

h) 6-Ethyl-9-Methyl-6,11-dihydro-5H-pyrido[2,3-b][1,5]benzodiazepin-5-on oder -thion; bei der Herstellung eines Arzneimittels zur Prophylaxe oder Behandlung eines dem HIV-1-Virus ausgesetzten oder davon infizierten Menschen.

**10.** Verbindung, ausgewählt aus der von den nachfolgenden Stoffen gebildeten Gruppe:

a) 2,4,6,8-Tetramethyl-6,11-dihydro-5H-pyrido[2,3-b][1,5]benzodiazepin-5-thion;
b) 6-Methyl-6,11-dihydro-5H-pyrido[2,3-b][1,5]benzodiazepin-5-thion;
c) 6,11-Dihydro-5H-pyrido[2,3-b][1,5]benzodiazepin-5-thion;
d) 6-Ethyl-6,11-dihydro-5H-pyrido[2,3-b][1,5]benzodiazepin-5-thion;
e) 6,8,9-Trimethyl-6,11-dihydro-5H-pyrido[2,3-b][1,5]benzodiazepin-5-thion;
f) 6-Ethyl-8,9-dimethyl-6,11-dihydro-5H-pyrido[2,3-b][1,5]benzodiazepin-5-thion;
g) 6-Isobutyl-6,11-dihydro-5H-pyrido[2,3-b][1,5]benzodiazepin-5-thion;
und
h) 6-Ethyl-9-Methyl-6,11-dihydro-5H-pyrido[2,3-b][1,5]benzodiazepin-5-thion; und pharmazeutisch annehmbare Salze davon.

**11.** Verfahren zur Herstellung einer Verbindung nach Anspruch 1 oder Anspruch 10, umfassend eines der nachfolgend angegebenen Verfahren:

A) Zur Herstellung einer Verbindung der Formel I, worin Z Sauerstoff bedeutet und $R^1$ und $R^2$ beide anderes bedeuten als Wasserstoff, Konversion eines 6,11-Dihydro-5H-pyrido[2,3-b][1,5]benzodiazepin-5-ons der Formel II

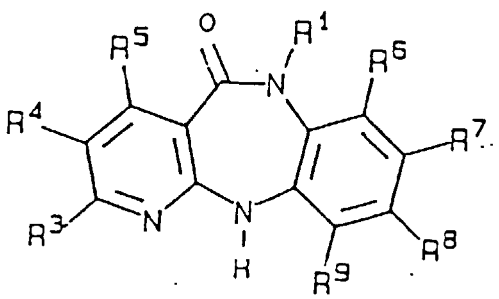

(II)

worin $R^1$ und $R^3$ bis $R^9$ die gleichen Bedeutungen haben wie in bezug auf Formel I definiert, jedoch $R^1$ anderes bedeutet als H, zur entsprechenden 11-Alkalimetallverbindung, und danach Umsetzung der Alkalimetallverbindung mit einer Verbindung der Formel III

$$R^2X \qquad \text{(III)}$$

worin $R^2$ die gleiche Bedeutung hat wie in bezug auf Formel I definiert, jedoch anderes bedeutet als Wasserstoff, und X ein geeigneter austretender Rest ist,

B) Zur Herstellung einer Verbindung der Formel I, worin Z Sauerstoff bedeutet und $R^1$ Wasserstoff bedeutet, Hydrolyse einer Verbindung der Formel IV

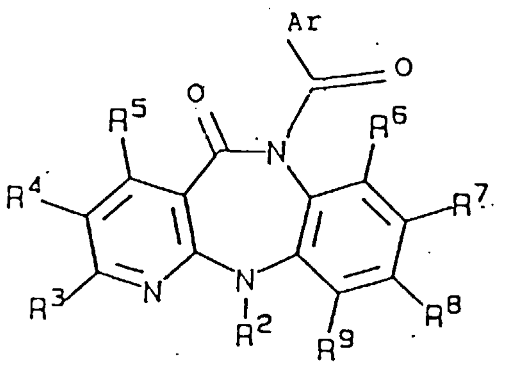

(IV)

worin die Reste $R^2$ bis $R^9$ die gleichen Bedeutungen haben wie in bezug auf Formel I definiert und Ar einen aromatischen oder heterocyclischen Rest bedeutet, der aus einem oder zwei Ringen besteht, der/die wahlweise mit Halogen, Methyl oder Methoxy substituiert ist/sind; oder

C) Zur Herstellung einer Verbindung nach Anspruch 10 oder eines 5-Thions der Formel I, Umsetzung einer 5-Thion-Verbindung, welche der gewünschten Verbindung des Anspruches 11 entspricht, oder einer 5-Thion-Verbindung der Formel I, mit einem Schwefelungsmittel, und danach Isolierung des resultierenden, nach den Methoden A, B oder C erhaltenen 5-Ons oder 5-Thions als solches oder als Säureadditionssalz.

**12.** Verfahren zur Herstellung eines Arzneimittels, umfassend das Mischen einer Verbindung nach Anspruch 1 oder Anspruch 10 mit einem pharmazeutisch annehmbaren Träger oder Arzneimittelträger.

**Revendications**

**1.** Composé de formule I

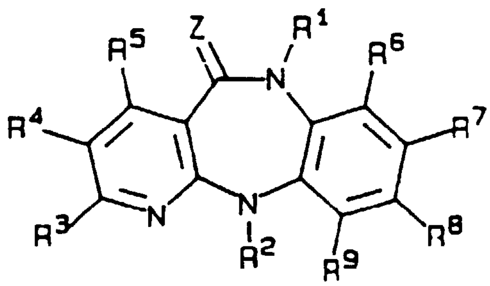

I

où

Z est l'oxygène ou le soufre,

$R^1$ est l'hydrogène, un groupe alkyle ou fluoroalkyle de 1 à 5 atomes de carbone, cyclopropyle, alcényle ou alcynyle de 2 à 5 atomes de carbone, 2-halo-propén-1-yle, arylméthyle (où la portion aryle est phényle, thiényle ou furanyle, qui est non substitué ou substitué par méthyle, méthoxy ou halogène), alcanoyle de 2 à 3 atomes de carbone, ou alcoxyalkyle ou alkylthioalkyle de 2 à 4 atomes de carbone,

$R^2$ est un groupe alkyle ou fluoroalkyle de 1 à 5 atomes de carbone, cycloalkyle de 3 à 5 atomes de carbone, alcényle ou alcynyle de 2 à 5 atomes de carbone, alcoxyalkyle ou alkylthioalkyle de 2 à 4 atomes de carbone, alcanoyle de 2 à 4 atomes de carbone, hydroxyalkyle de 2 à 5 atomes de carbone, arylméthyle (où la portion aryle est phényle, thiényle ou furanyle, qui est non substitué ou substitué par alkyle ou alcoxy de 1 à 3 atomes de carbone, hydroxyle ou halogène), phényle (qui est non substitué ou substitué par alkyle ou alcoxy de 1 à 3 atomes de carbone, halogène ou hydroxyle) ou alcoxycarbonylméthyle où la portion alcoxy contient 1 à 5 atomes de carbone,

$R^3$, $R^4$ et $R^5$ sont chacun indépendamment l'hydrogène ou un groupe alkyle de 1 à 3 atomes de carbone, avec la condition qu'au moins l'un de ces substituants soit l'hydrogène, ou l'un des substituants $R^3$, $R^4$ et $R^5$ est un groupe butyle, alcanoyle de 1 à 3 atomes de carbone, hydroxyalkyle de 1 à 4 atomes de carbone, alcoxycarbonyle de 2 à 3 atomes de carbone, alcoxycarbonylalkyle où les portions alcoxy et alkyle contiennent l'une et l'autre 1 à 2 atomes de carbone, halogène, trihalométhyle, hydroxyle, alcoxy de 1 à 3 atomes de carbone, alkylthio de 1 à 3 atomes de carbone, alcanoyloxy de 2 à 3 atomes de carbone, alcanoylamino de 1 à 3 atomes de carbone, aminoalkyle de 1 à 3 atomes de carbone, mono- ou dialkylamino où chaque portion alkyle contient 1 à 2 atomes de carbone, carboxyalkyle de 2 à 3 atomes de carbone, cyano, nitro, carboxyle, carbamyle, amino, azido ou mono- ou dialkylaminoalkyle où les portions alkyle contiennent chacune 1 à 2 atomes de carbone, et les deux substituants restants sont l'hydrogène ou méthyle, ou lorsque Z est l'oxygène, l'un des substituants $R^3$, $R^4$ et $R^5$ est un groupe alkylsulfinyle ou alkylsulfonyle de 1 à 3 atomes de carbone, avec la condition que les deux substituants restants soient l'hydrogène ou méthyle, et

$R^6$, $R^7$, $R^8$ et $R^9$ sont chacun l'hydrogène, ou l'un des substituants $R^6$, $R^7$, $R^8$ et $R^9$ est un groupe alkyle de 1 à 4 atomes de carbone, alcanoyle de 1 à 3 atomes de carbone, alcoxycarbonyle de 2 à 3 atomes de carbone, hydroxyalkyle de 1 à 4 atomes de carbone, alcoxycarbonylalkyle où les portions alcoxy et alkyle contiennent l'une et l'autre 1 à 2 atomes de carbone, halogène, trihalométhyle, hydroxyle, alcoxy de 1 à 3 atomes de carbone, alkylthio de 1 à 3 atomes de carbone, alcanoyloxy de 2 à 3 atomes de carbone, alcanoylamino de 1 à 3 atomes de carbone, aminoalkyle de 1 à 3 atomes de carbone, mono- ou dialkylamino où chaque portion alkyle contient 1 à 2 atomes de carbone, carboxylalkyle de 2 à 3 atomes de carbone, cyano, nitro, carboxyle, carbamyle, amino, azido ou mono- ou dialkylaminoalkyle où chaque portion alkyle contient 1 à 2 atomes de carbone, et les trois substituants restants sont l'hydrogène ou deux des trois substituants restants sont l'hydrogène et l'un est méthyle, éthyle ou halogène,

ou sel d'addition d'acide pharmaceutiquement acceptable de celui-ci.

**2.** Composé de formule I selon la revendication 1 où

Z est l'oxygène ou le soufre,

$R^1$ est l'hydrogène, alkyle de 1 à 4 atomes de carbone, alcényle ou alcynyle de 2 à 4 atomes de carbone, 2-halopropén-1-yle, ou alcoxyalkyle ou alkylthioalkyle de 2 à 3 atomes de carbone,

$R^2$ est alkyle de 1 à 4 atomes de carbone, cycloalkyle de 3 à 4 atomes de carbone, alcényle ou alcynyle de 2 à 4 atomes de carbone, alcoxyalkyle ou alkylthioalkyle de 2 à 4 atomes de carbone, alcanoyle de 2 à 3 atomes de carbone, hydroxyalkyle de 2 à 4 atomes de carbone, arylméthyle (où la portion aryle est phényle ou thiényle, qui est non substitué ou substitué par méthyle, méthoxy, hydroxyle ou halogène), phényle (qui est non substitué ou substitué par méthyle, méthoxy, hydroxyle ou halogène) ou alcoxycarbonylméthyle où la portion alcoxy contient 1 à 5 atomes de carbone,

$R^3$, $R^4$ et $R^5$ sont chacun indépendamment l'hydrogène ou méthyle, avec la condition qu'au moins 1' un de ces substituants soit l'hydrogène, ou $R^5$ est éthyle, propyle ou butyle, les deux autres substituants étant l'hydrogène,

$R^6$ est l'hydrogène, méthyle ou éthyle avec la condition que $R^7$ soit l'hydrogène ou méthyle,

$R^7$ est alkyle de 1 à 3 atomes de carbone, alcanoyle de 1 à 3 atomes de carbone, alcoxycarbonyle de 1 à 3 atomes de carbone, hydroxyalkyle de 1 à 3 atomes de carbone, alcoxycarbonylalkyle où les portions alcoxy et alkyle contiennent chacune 1 à 2 atomes de carbone, halogène, trifluorométhyle, hydroxyle, alcoxy ou alkylthio de 1 à 2 atomes de carbone, acétyloxy, alcanoylamino ou aminoalkyle de 1 à 2 atomes de carbone, cyano, nitro, amino, ou mono- ou diméthyl- ou -éthylamino, avec la condition que $R^8$ soit l'hydrogène,

$R^8$ est alkyle de 1 à 3 atomes de carbone, alcanoyle de 1 à 3 atomes de carbone, alcoxycarbonyle de 1 à 3 atomes de carbone, hydroxyalkyle de 1 à 3 atomes de carbone, alcoxycarbonylalkyle où les portions alcoxy et alkyle contiennent chacune 1 à 3 atomes de carbone, halogène, trifluorométhyle, hydroxyle, alcoxy ou alkylthio de 1 à 2 atomes de carbone, acétyloxy, alcanoylamino ou aminoalkyle de 1 à 2 atomes de carbone, cyano, nitro, amino, ou mono- ou diméthyl- ou -éthylamino avec la condition que $R^7$ soit l'hydrogène, ou lorsque Z est l'oxygène et $R^8$ est l'hydrogène ou méthyle, $R^7$ peut être en outre alkylsulfinyle ou alkylsulfonyle de 1 à 2 atomes de carbone, et lorsque Z est l'oxygène et $R^7$ est l'hydrogène ou méthyle, $R^8$ peut être en outre alkylsulfinyle ou alkylsulfonyle de 1 à 2 atomes de carbone, ou

$R^7$ et $R^8$ sont l'un est l'autre l'hydrogène, méthyle ou halogène, et

$R^9$ est l'hydrogène ou méthyle, avec la condition que $R^8$ soit l'hydrogène ou méthyle,

ou sel d'addition d'acide pharmaceutiquement acceptable de celui-ci.

**3.** Composé de formule I selon la revendication 1 où Z est l'oxygène ou le soufre,

$R^1$ est l'hydrogène, alkyle de 1 à 3 atomes de carbone, 2-halo-2-propén-1-yle, ou alcoxyalkyle ou alkylthioalkyle de deux à trois atomes de carbone,
$R^2$ est alkyle de 1 à 4 atomes de carbone, cycloalkyle de 3 à 4 atomes de carbone, alcényle ou alcynyle de 2 à 4 atomes de carbone, alkyloxyalkyle ou alkylthioalkyle de 2 à 3 atomes de carbone, alcanoyle de 2 à 3 atomes de carbone, hydroxyalkyle de 2 à 4 atomes de carbone, arylméthyle (où la portion aryle est phényle ou thiényle, qui est non substitué ou substitué par méthyle, méthoxy ou halogène) ou alcoxycarbonylméthyle où la portion alcoxy contient 1 à 5 atomes de carbone, et
$R^3$ à $R^9$ sont tels que définis ci-dessous dans le tableau A, ou sel d'addition d'acide pharmaceutiquement acceptable de celui-ci

TABLEAU A

| | $R^3$ | $R^4$ | $R^5$ | $R^6$ | $R^7$ | $R^8$ | $R^9$ |
|---|---|---|---|---|---|---|---|
| a | H | H | H | H | H | $CF_3$ | H |
| b | H | H | H | H | Cl | H | H |
| c | H | H | H | H | $CH_3$ | $CH_3$ | H |
| d | $CH_3$ | H | H | H | $CH_3$ | $CH_3$ | H |
| e | $CH_3$ | $CH_3$ | H | H | $CH_3$ | $CH_3$ | H |
| f | H | H | H | H | $CH_3$ | $CH_3$ | H |
| g | H | H | H | H | H | Cl | H |
| h | H | H | H | H | H | H | H |
| i | H | H | H | $CH_3$ | H | H | H |
| j | H | H | H | H | $CH_3O_2C$- | H | H |
| k | H | H | H | H | $C_2H_5O_2C$- | H | H |
| l | H | H | H | H | NC- | H | H |
| m | H | H | H | H | $CH_3CO$- | H | H |
| n | H | H | H | H | H | $CH_3O_2C$- | H |
| o | H | H | H | H | H | $C_2H_5O_2C$- | H |
| p | H | H | H | H | H | NC- | H |
| q | H | H | H | H | H | $CH_3CO$- | H |

**4.** Composé de formule I selon la revendication 1 où Z est l'oxygène ou le soufre,

$R^1$ est l'hydrogène, alkyle de 1 à 2 atomes de carbone ou allyle,
$R^2$ est alkyle de 2 à 3 atomes de carbone, cyclopropyle ou allyle, et
$R^3$ à $R^9$ sont chacun l'hydrogène ou $R^7$ et $R^8$ sont l'un et l'autre méthyle ou chloro et $R^3$, $R^4$, $R^5$, $R^6$ et $R^9$ sont chacun l'hydrogène,

ou sel pharmaceutiquement acceptable de celui-ci.

**5.** Composé de formule I selon la revendication 1 choisi dans le groupe consistant en les suivants :

a) 6,11-dihydro-11-éthyl-6-méthyl-5H-pyrido[2,3-b][1,5]benzodiazépin-5-one,
b) 6,11-dihydro-6-méthyl-11-propyl-5H-pyrido[2,3-b][1,5]benzodiazépin-5-one,
c) 6,11-dihydro-6-méthyl-11-méthyléthyl-5H-pyrido[2,3-b][1,5]benzodiazépin-5-one,
d) 6,11-dihydro-11-éthyl-6-méthyl-5H-pyrido[2,3-b] [1,5]benzodiazépin-5-one,
e) 6,11-dihydro-11-éthyl-5H-pyrido[2,3-b] [1,5]benzodiazépin-5-one,
f) 6,11-dihydro-11-éthyl-5H-pyrido[2,3-b] [1,5]benzodiazépine-5-thione,
g) 6,11-dihydro-6,8,9,11-tétraméthyl-5H-pyrido[2,3-b] [1,5]benzodiazépin-5-one,
h) 6,11-dihydro-6-éthyl-11-méthyl-5H-pyrido[2,3-b][1,5]benzodiazépin-5-one,
i) 6,11-dihydro-6-n-propyl-11-méthyl-5H-pyrido[2,3-b] [1,5]benzodiazépin-5-one,
j) 6,11-dihydro-6-i-propyl-11-méthyl-5H-pyrido[2,3-b] [1,5]benzodiazépin-5-one,
k) 6,11-dihydro-6,11-diéthyl-5H-pyrido[2,3-b][1,5]benzodiazépin-5-one,
l) 6,11-dihydro-11-éthyl-5H-pyrido[2,3-b] [1,5]benzodiazépine-5-thione,

m) 6,11-dihydro-11-éthyl-6,8,9-triméthyl-5H-pyrido[2,3-b][1,5]benzodiazépin-5-one,
n) 6,11-dihydro-8-méthoxycarbonyl-11-éthyl-6-méthyl-5H-pyrido[2,3-b][1,5]benzodiazépin-5-one et
e) 6,11-dihydro-11-fluoroéthyl-6-méthyl-5H-pyrido[2,3-b][1,5]benzodiazépin-5-one.

**6.** 6,11-dihydro-11-éthyl-6-méthyl-5H-pyrido[2,3-b][1,5]benzodiazépin-5-one.

**7.** Utilisation d'un composé selon les revendications 1, 2, 3, 4, 5 ou 6 dans la fabrication d'une préparation pharmaceutique pour la prophylaxie ou le traitement d'un humain exposé à ou infecté par VIH-1.

**8.** Composition pharmaceutique pour la prévention ou le traitement d'une infection à VIH-1 comprenant un composé selon l'une quelconque des revendications 1, 2, 3, 4, 5 ou 6 et un support pharmaceutiquement acceptable.

**9.** Utilisation d'un composé choisi dans le groupe consistant en :

a) la 2,4,6,8-tétraméthyl-6-11-dihydro-5H-pyrido[2,3-b][1,5]benzodiazépin-5-one ou -thione;
b) la 6-méthyl-6,11-dihydro-5H-pyrido[2,3-b] [1,5]benzodiazépin-5-one ou -thione;
c) la 6-11-dihydro-5H-pyrido[2,3-b] [1,5]benzodiazépin-5-one ou -thione;
d) la 6-éthyl-6,11-dihydro-5H-pyrido[2,3-b] [1,5]benzodiazépin-5-one ou -thione;
e) la 6,8,9-triméthyl-6,11-dihydro-5H-pyrido[2,3-b][1,5]benzodiazépin-5-one ou -thione;
f) la 6-èthyl-8,9-diméthyl-6,11-dihydro-5H-pyrido[2,3-b][1,5]benzodiazépin-5-one ou -thione;
g) la 6-isobutyl-6,11-dihydro-5H-pyrido[2,3-b][1,5]benzodiazépin-5-one ou -thione;
et
h) la 6-éthyl-9-méthyl-6,11-dihydro-5H-pyrido[2,3-b] [1,5]benzodiazépin-5-one ou -thione

dans la fabrication d'une préparation pharmaceutique pour la prophylaxie ou le traitement d'un humain exposé à ou infecté par VIH-1.

**10.** Composé choisi dans le groupe consistant en les suivants :

a) la 2,4,6,8-tétraméthyl-6-11-dihydro-5H-pyrido[2,3-b][1,5]benzodiazépine-5-thione;
b) la 6-méthyl-6,11-dihydro-5H-pyrido [2, 3-b] [1,5]benzodiazépine-5-thione;
c) la 6-11-dihydro-5H-pyrido[2,3-b][1,5]benzodiazépine-5-thione;
d) la 6-éthyl-6,11-dihydro-5H-pyrido[2,3-b] [1,5]benzodiazépine-5-thione;
e) la 6,8,9-triméthyl-6,11-dihydro-5H-pyrido[2,3-b] [1,5]benzodiazépine-5-thione;
f) la 6-éthyl-8,9-diméthyl-6,11-dihydro-5H-pyrido[2,3-b][1,5]benzodiazépine-5-thione;
g) la 6-isobutyl-6,11-dihydro-5H-pyrido[2,3-b] [1,5]benzodiazépine-5-thione;
et
h) la 6-éthyl-9-méthyl-6,11-dihydro-5H-pyrido[2,3-b] [1,5]benzodiazépine-5-thione
et leurs sels pharmaceutiquement acceptables.

**11.** Procédé pour la préparation d'un composé tel que défini dans la revendication 1 ou la revendication 10, comprenant l'un des procédés suivants

A) pour préparer un composé de formule 1 où Z est l'oxygène et $R^1$ et $R^2$ sont l'un et l'autre différents de l'hydrogène, convertir une 6,11-dihydro-5H-pyrido-[2,3-b][1,5]benzodiazépin-5-one de formule II

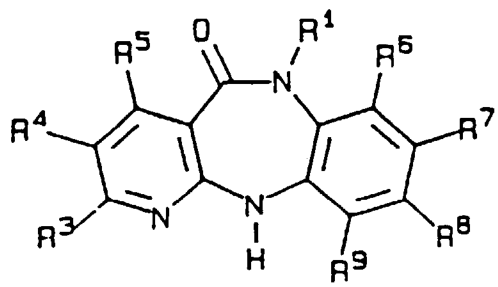

II

où $R^1$ et $R^3$ à $R^9$ ont les mêmes significations que celles indiquées concernant la formule 1 mais $R^1$ est différent de H, en le composé 11-métal alcalin correspondant, puis faire réagir le composé de métal alcalin avec un composé de formule III

$$R^2X \qquad III$$

où $R^2$ a les mêmes significations que celles indiquées concernant la formule 1 mais est différent de l'hydrogène, et X est un groupe partant approprié,

B) pour préparer un composé de formule 1 où Z est l'oxygène et $R^1$ est l'hydrogène, hydrolyser un composé de formule IV

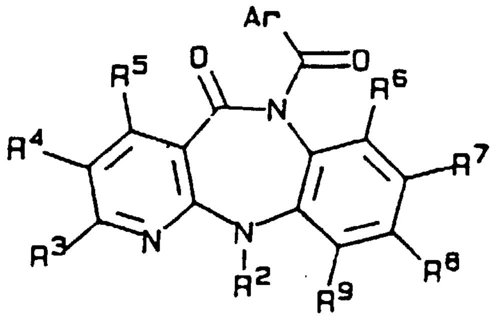

IV

où les groupes $R^2$ à $R^9$ sont tels qu'indiqués concernant la formule 1 et Ar est un groupe aromatique ou hétérocyclique consistant en un ou deux noyaux qui est ou sont éventuellement substitués par halogène, méthyle ou méthoxy, ou

C) pour préparer un composé de formule 10 ou une 5-thione de formule I, faire réagir un composé 5-one correspondant en le composé selon la revendication 11 voulu, ou un composé 5-one de formule I avec un agent de sulfuration, puis isoler la 5-one ou 5-thione obtenue par le procédé A, B ou C en l'état ou sous forme d'un sel d'addition d'acide.

**12.** Procédé pour la préparation d'une composition pharmaceutique qui comprend le mélange d'un composé selon la revendication 1 ou la revendication 10 avec un support ou excipient pharmaceutiquement acceptable.